(19) Europäisches Patentamt
European Patent Office
Office européen des brevets

(11) **EP 4 578 401 A1**

(12) **EUROPEAN PATENT APPLICATION**

(43) Date of publication:
**02.07.2025 Bulletin 2025/27**

(21) Application number: **23220682.1**

(22) Date of filing: **29.12.2023**

(51) International Patent Classification (IPC):
*A61B 7/00* (2006.01)    *A61B 5/00* (2006.01)
*G16H 50/20* (2018.01)

(52) Cooperative Patent Classification (CPC):
**A61B 7/008; A61B 5/392; A61B 5/4216;
A61B 5/6804; A61B 5/7267; G16H 50/20;
G16H 50/70;** A61B 5/725; A61B 2562/0204

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Hahn-Schickard-Gesellschaft
für angewandte Forschung e. V.
70569 Stuttgart (DE)**

(72) Inventors:
• **Baronetto, Annalisa
91052 Erlangen (DE)**
• **Amft, Oliver
4312 Magden (CH)**

(74) Representative: **Hertin und Partner
Rechts- und Patentanwälte PartG mbB
Kurfürstendamm 63
10707 Berlin (DE)**

(54) **AUTOMATED INFLAMMATORY BOWEL DISEASE DETECTION USING BOWEL SOUNDS MONITORED BY A WEARABLE SYSTEM**

(57)    The invention relates to a method for assisting the diagnosis of a bowel disease comprising the steps of: a) providing an audio-recording of sounds emitted by the bowel of a subject for the duration of at least one classification time window, b) analyzing the processed audio data thereby classifying at least a fraction of the recorded sounds as bowel sound, c) computing at least one Mel Frequency Cepstral Coefficient (MFCC)-feature from at least a fraction of the recorded bowel sounds extracted in b), d) determining at least one statistical parameter, preferably the mean and/or variance, of at least a fraction of the Mel Frequency Cepstral Coefficient (MFCC)-features computed in c) within at least one classification time window, e) using an artificial intelligence (AI) to classify the at least one classification time window as either being indicative for the individual suffering from a bowel disease or for a healthy individual, wherein the AI is taking into account the in step d) determined at least one statistical parameter of at least a fraction of the MFCC-features, thereby predicting the likelihood of a bowel disease for the subject. The invention further relates to the use of the present method for the diagnosis of a bowel disease, to a computer-implemented method and a software or computer program for predicting the likelihood of a bowel disease in a subject.

Fig. 7

**Description**

The invention relates to a method for assisting the diagnosis of a bowel disease comprising the steps of: a) providing an audio-recording of sounds emitted by the bowel of a subject for the duration of at least one classification time window, b) analyzing the processed audio data thereby classifying at least a fraction of the recorded sounds as bowel sound, c) computing at least one Mel Frequency Cepstral Coefficient (MFCC)-feature from at least a fraction of the recorded bowel sounds extracted in b), d) determining at least one statistical parameter, preferably the mean and/or variance, of at least a fraction of the Mel Frequency Cepstral Coefficient (MFCC)-features computed in c) within at least one classification time window, e) using an artificial intelligence (AI) to classify the at least one classification time window as either being indicative for the individual suffering from a bowel disease or for a healthy individual, wherein the AI is taking into account the in step d) determined at least one statistical parameter of at least a fraction of the MFCC-features, thereby predicting the likelihood of a bowel disease for the subject. The invention further relates to the use of the present method for the diagnosis of a bowel disease and to a computer-implemented method and a software or computer program for predicting the likelihood of a bowel disease in a subject.

**BACKGROUND OF THE INVENTION**

Inflammatory Bowel Diseases (IBD) are chronic conditions that reduce patients' quality of life. The two most common forms of IBD, Ulcerative Colitis (UC) and Chron's Disease (CD), cause inflammation at specific locations of the gastrointestinal (GI) tract, resulting in symptoms such as abdominal pain, diarrhea, rectal bleeding, and weight loss [1]. IBD patients often experience periods of disease remission and relapse, requiring thus regular hospital visits. According to IBD epidemiology, UC and CD primarily affect adolescents and young adults [2]. Overall, it was estimated that 0.3% of European population was diagnosed with IBD [3]. Similarly, North America reported a prevalence of 0.5% of the population. While IBD is mostly prevalent in western countries, recent studies reported increasing incidence in eastern and developing countries [3]. IBD incidence is associated with lifestyle and environment of industrialized countries, nevertheless root causes remain unclear to date.

To determine IBD conditions, various diagnostic investigations are recommended [4]. Disease activity can be initially assessed with questionnaires about patient symptoms, e.g. Harvey-Bradshaw Index for CD and Mayo Score for UC. Guidelines [4] suggest to further examine the patient by analyzing relevant biomarkers collected from blood and stool samples. C-reactive protein (CRP) and leukocytes are blood biomarkers that indicate acute inflammation, nevertheless they are not IBD-specific. In contrast, the stool biomarker fecal Calprotectin (fCP) is more sensitive to intestinal inflammation. Furthermore, fCP is correlated with endoscopic and histological assessment of IBD [5], which is considered to be the reference diagnostic tool. Because fCP information cannot be used to distinguish IBD from other GI conditions, an endoscopic evaluation is still recommended in the early stages of diagnosis [4]. However, endoscopic procedures require long bowel preparation, cause patient discomfort despite the sedation, and are expensive [4, 6]. Moreover, endoscopic evaluation can be affected by high inter-observer variability [7]. Imaging techniques, e.g., ultrasounds or radiology, are also employed to locate inflammation to evaluate mucosal inflammation at GI regions that are inaccessible with endoscopy. Nevertheless, imaging diagnostics often require long scan time or radiation exposure [8], thus limiting its applicability. Therefore, IBD diagnosis still relies nowadays on a combination of mostly invasive examinations, which challenges diagnostic efforts.

Over the last decades, Artificial Intelligence (AI)-based methods were proposed to support IBD diagnosis and treatment. Various AI-based models were tested to diagnose as well as predict risk of IBD [9]. For instance, Han et al. [10] analyzed the performance of a Random Forest Classifier in detecting IBD using genetic features extracted with probabilistic graphical models. On five datasets containing tissue samples from CD/UC patients and healthy controls, the median Area Under the Receiver Operating Characteristic curve (AUROC) varied between 0.6 and 0.76 on the validation data. Chierici et al. [11] trained ResNet neural networks on endoscopic images to identify pathological samples. The authors reported sensitivity and specificity above 0.90, when detecting pathological images vs. control. The models were also tested to distinguish UC from CD, achieving sensitivity of 0.90 and specificity > 0.75. Stidham et al. [12] proposed and trained an inception neural network to assess UC disease severity from endoscopic images. The model assessment was compared against Mayo Scores assigned by human experts, and achieved 0.83 sensitivity and 0.96 specificity when classifying between remission and moderate-to-severe disease states. However, the aforementioned works required expensive and invasive techniques to collect patient data, which could limit their application in clinical practice.

Among the various techniques available to inspect the abdomen, auscultation, i.e. listening to body sounds with a stethoscope, is inexpensive and non-invasive. Manual auscultation of Bowel Sounds (BS) using a stethoscope had been introduced by Läennec in 18th century and is today a standard clinical practice performed during preliminary examinations, conducted for typically a few minutes [13, 14]. Reduced amount of BS could indicate bowel obstruction or paralytic ileus, while IBD or gastroenteritis could result in more frequent BS occurrences. However, BS-based clinical assessment remains controversial to date due to the rather qualitative, manual evaluation of BS and the lack of BS acoustic properties quantification [15]. Previous investigations showed already that BS characteristics could support the diagnosis of GI disorders, e.g. by detecting abnormal BS patterns [16]. For example, Craine et al. [17] collected and compared BS from patients with Irritable Bowel Syndrome (IBS) and healthy controls. Their statistical analysis showed that sound-to-sound (SS) time interval between consecutive BS was significantly different across the study groups when recording BS with emptied stomach (fasting phase). Consequently, the authors proposed a threshold-based algorithm to detect IBS. In a later study [18], the authors included BS recorded from CD patients and suggested that further SS time interval ranges should be used to discriminate IBS and CD. However, their analysis was conducted on short, 2-minute audio recordings. Further shortcomings of the method were the requirement of optimal skin-sensor contact of the electronic stethoscope used and that the approach was based on the sound-to-sound interval profile of bowel sounds, which did not assure a high reliability of diagnosis and suffered from low applicability in clinical practice.

While manual auscultation is usually performed for a few minutes only, the diagnosis of abdominal pain based on BS information could be challenging, as reported by Ranta et al [19]. Due to the irregular BS occurrences and varying abdominal location, the authors suggested to extend the BS observation period.

Du et al. [20] included in their analysis over 2 h of audio recorded across IBS patients and healthy participants. The authors investigated various BS acoustic features and used logistic regression o diagnose IBS, achieving 0.90 sensitivity and 0.92 specificity on leave-one-out Cross-Validation (CV) experiments.

In WO 2019/157552 A1 Marshall et al. describe a computer-implemented system for determining the likelihood of a gastrointestinal condition by analyzing bowel sounds. However, this method requires at minimum more than two and a half hours of audio recording for a successful calculation of a likelihood of a gastrointestinal condition. Moreover, multiple sensors are required to record and analyze bowel sounds. This constitutes a significant hurdle for the practical applicability of said method.

Spiegel et al. [21] designed a disposable wearable sensor embedding a microphone and recorded BS from postoperative patients tolerating feeding, patients with postoperative ileus (POI), and healthy controls. The analysis of BS event temporal frequency showed that POI and non-POI patient groups were statistically different. Although those previous studies showed that BS-derived GI information could be exploited to predict GI disorders, none of the works focused on IBD detection using BS acoustic features.

In light of the prior art there remains a significant need in the art to provide additional means for the reliable, time-efficient and straight forward methods for the diagnosis of bowel diseases, such as IBD.

## SUMMARY OF THE INVENTION

In light of the prior art the technical problem underlying the present invention is to provide improved methods that are able to assist clinicians in the diagnosis of bowel diseases, such as IBD.

This problem is solved by the features of the independent claims. Preferred embodiments of the present invention are provided by the dependent claims.

The invention therefore relates to a method for assisting the diagnosis of a bowel disease comprising the steps of:

a) providing an audio-recording of sounds emitted by the bowel of a subject for the duration of at least one classification time window,

b) analyzing the processed audio data thereby classifying at least a fraction of the recorded sounds as bowel sound,

c) computing at least one Mel Frequency Cepstral Coefficient (MFCC)-feature from at least a fraction of the recorded bowel sounds extracted in b),

d) determining at least one statistical parameter, preferably the mean and/or variance, of at least a fraction of the Mel Frequency Cepstral Coefficient (MFCC)-features computed in c) within at least one classification time window,

e) using an artificial intelligence (AI) to predict a likelihood of a bowel disease for (in) the subject from at least one classification time window, wherein the AI is taking into account the in step e) determined at least one statistical parameter of at least a fraction of the MFCC-features.

In the context of the present invention one specific Mel Frequency Cepstral Coefficient (MFCC) may be regarded as one specific feature. In other words, MFCC are preferably one type of feature that may be assessed/considered in the context of the present method. Hence, the terms 'MFCC-feature' and simply 'MFCC' may be used herein interchangeably.

In embodiments, the bowel disease may be selected from Inflammatory Bowel Disease (IBD), Irritable Bowel Syndrome (IBS), diverticulitis, celiac disease and other functional bowel disorders.

In embodiments, functional bowel disorders (FBD) (functional gastrointestinal disorders) include Irritable bowel syndrome (IBS), functional bloating, functional constipation, and functional diarrhea.

In embodiments inflammatory bowel diseases comprise Crohn's disease and ulcerative colitis.

The diagnosis and monitoring of bowel diseases and conditions e.g., IBD, relies nowadays on a combination of clinical, imaging and biochemical assessments. Due to the variety of investigations needed and, consequently, the time required to perform the investigations, bowel disease diagnosis is time intensive and often invasive. Inflammation-biomarkers, are rarely specific for certain diseases, e.g., IBD or IBS, such that more invasive techniques are performed to confirm the diagnosis, e.g. endoscopy.

Although endoscopy is considered the gold standard to ultimately diagnose bowel diseases, e.g., IBD or IBS, as well as assess disease state, the technique is costly, invasive, and can cause patient discomfort.

The inventors herein aimed to provide physicians a new approach to non-invasively detect bowel diseases, such as IBS or IBD, from continuous abdominal recordings. The inventors investigated bowel sound (BS)-derived features and trained an artificial intelligence to make predictions from short audio recordings captured from IBD patients vs. healthy.

Several prior art methods are concerned with the computational evaluation of bowel sounds, however, most of them are not practicable in a daily clinical routine and none of them achieves results that are as reproducible and reliable as the analysis of bowel sounds by an expert through auscultation. The inventors considered that the calculation of Mel Frequency Cepstral Coefficients (MFCC) and features thereof from audio recordings of a patients abdomen may improve the reliability and reproducibility of the identification of bowel sound events in an audio recording that are indicative for a bowel disease.

As shown by the examples, the present method has the advantages, that a particularly reliable and reproducible prognosis / likelihood that the analyzed sounds were recorded from a subject suffering from a bowel disease may be provided. Notably to this end only a short time windows (e.g. as short as 10 minutes) of audio recording need to be analyzed. This constitutes a significant advantage and improvement of the methods of the prior art. One of the features ensuring this effect is the calculation of Mel Frequency Cepstral Coefficients (MFCC) for the audio data analysis.

In the present examples, a low-cost wearable device was used to collect BS across different digestive phases inconspicuously. Compared to existing diagnostics, the present approach is inexpensive, easy to use and can monitor digestion throughout the whole process, avoiding repeated and uncomfortable abdominal assessments. The inventors consider that the present method can be employed as a screening test for bowel diseases, such as IBD, and can be used as a telemedicine solution, minimizing thus the amount of patient hospital visits.

The inventors investigated relevant bowel sound (BS) features to acoustically detect IBD in continuous abdominal recordings. The experiments disclosed in the present examples included audio abdominal recordings from 24 IBD patients with various disease activity and 21 healthy participants with no gastrointestinal (GI) disorders. BS events (bowel sounds identified as one sound event, that is distinguishable/separable of other sounds) were marked on the recordings of 27 participants in the data streams by pairs of expert raters and the manual labels were used to train a deep-learning BS pattern spotting algorithm (an algorithm that identifies single bowel sound events).

In embodiments resampling is applied to training data to minimize data imbalance. In embodiments a data training set of each k fold may be resampled, e.g., using SMOTE (Synthetic Minority Oversampling Technique), to balance the amount of classification windows extracted from patients suffering from a bowel disease (e.g., IBD patients) and optionally healthy controls.

In embodiments resampling is applied to training data to minimize data imbalance using resampling methods, such as SMOTE, ADASYN or random oversampling.

The inventors used BS features to train a Gradient Boosting Classifier (GBC) to predict IBD vs. healthy controls. Their approach achieved a mean AU-ROC above 0.83 regardless of manual or spotting-based BS retrieval. The area under the receiver operating characteristic (AUC - ROC curve ) curve is a metric for the performance of a classification and visualizes if a computational model (e.g., an algorithm or machine learning model) is capable of distinguishing between two different classes (here bowel disease or heathy), wherein a high AUC shows that a model is reliably distinguishing between patients with a bowel disease and no disease. The present model shows therefore a surprisingly good performance in identifying bowel sounds, e.g., from an individual suffering from IBD, from an audio recording.

As the long time span of audio recordings that are required by prior art methods for the estimation of the presence of a bowel disease significantly impairs their practicability in the clinics, the inventors further analyzed the minimum audio recording duration required by their present method to accurately detect bowel diseases, such as IBS or IBD, and surprisingly found, that the present method enables reliable predictions regarding the presence of a bowel disease after the analysis of audio recordings as short as 10 minutes. However, as can be expected, longer recordings than the common manual auscultation duration yielded even higher AUROC. The inventors aimed to develop a method that can support clinicians in IBD diagnosis during the initial screening stages. Their analysis on correlation between IBD classification and inflammation biomarkers showed that BS features could be related with disease activity and, therefore, further employed in disease activity assessment in IBD treatment and monitoring.

In embodiments step e) of the present method comprises using an artificial intelligence (AI) to classify the at least one classification time window as either being indicative for the individual suffering from a bowel disease or for a healthy

individual, wherein the AI is taking into account the in step d) determined at least one statistical parameter of at least a fraction of the MFCC-(feature)s, thereby predicting the likelihood of a bowel disease or a score indicative for the existence of a bowel disease for the subject. Hence, in embodiments the likelihood of a bowel disease is indicated by a (numerical) score indicative for the existence a bowel disease in the subject.

In embodiments, depending on the AI used, the output could be a score, e.g., a bowel disease prediction score, or likelihood. If a Bayesian approach is employed in the present method in step e), likelihood could also be probability.

In embodiments, the likelihood may be expressed as a numerical ((bowel disease) prediction) score, wherein 0 indicates the lowest possible likelihood of a bowel disease (exclusion) and 1 indicates the highest likelihood of a bowel disease. In embodiments, a high likelihood over 50% or over 0.5 may be indicative of the presence of a bowel disease in the subject and may indicate to a physician or medical professional to prescribe further diagnostic tests (e.g., more invasive tests) to confirm the presence of a bowel disease in the subject. Subsequent diagnostic test may be biopsies and/or colonoscopy.

The present examples show that the present method is able to determine a bowel disease probability of > 0.50 for all patients, regardless of the disease condition. In particular, IBD was predicted by GBC with accuracy 0.91. The results show that this method could be potentially employed in clinical practice as a gastrointestinal disease, such as IBD or IBS, screening test prior to use or instead of more invasive investigations such as endoscopy.

In embodiments, a first step comprises the determination/recording of a continuous audio recording (using one or more microphones) of the abdomen of a subject, e.g., during digestion. In embodiments, the determination/recording of a continuous audio recording of the abdomen of a subject may also be referred to as bowel sound (BS) data collection.

In embodiments, preferably a one-channel analysis is performed. In other embodiments a multi-channel analysis is performed. While a one-channel analysis is in embodiments a sufficient and simple solution to detect bowel disease, a multi-channel analysis may in other embodiments be employed alternatively, to locate the gastro-intestinal section affected by the bowel disease, e.g., inflamed regions in case of IBD, or improve classification performance.

Herein, as sound annotated or classified as a (separate/single/distinguishable) bowel sound may also be referred to as 'bowel sound event' or 'BS event'.

In embodiments, the classification may be achieved either manually (e.g., evaluation and annotation by an expert (e.g., gastroenterologist)) or with an artificial intelligence (AI) model (e.g., an Efficient-U-Net).

In embodiments, the bowel sound classification ('BS spotting') may be achieved employing an Efficient-U-Net.

In embodiments, the annotation (or classification/identification) of bowel sounds in step b) is performed using an artificial intelligence (AI, or machine learning (model)) and/or via manual annotation by an expert.

In some embodiments, a bowel sound event (a single/separate bowel sound; BS event) may (also) be annotated/classified/identified by an artificial intelligence (AI, or machine learning (model)) and/or manual annotation by an expert on the basis of its duration, e.g., wherein the duration of a bowel sound may be pre-defined/pre-set to be $\geq 5$ ms, $\geq 10$ ms, $\geq 15$ ms, $\geq 18$ ms, $\geq 20$ ms, $\geq 30$ ms, $\geq 40$ ms, $\geq 60$ ms or $\leq 5$ ms, $\leq 10$ ms, $\leq 15$ ms, $\leq 18$ ms, $\leq 20$ ms, $\leq 30$ ms, $\leq 40$ ms, $\leq 60$ between 1-30 ms, or between 1-20 ms. In some embodiments the duration of a bowel sound is pre-defined/pre-set to be $\geq 18$ ms. In some embodiments, a bowel sound event may be classified/identified as a single BS event, if the sound-to-sound interval of consecutive bowel sounds lie with < 100 ms.

In embodiments, the recording is subjected to audio preprocessing. In embodiments said audio preprocessing may comprise applying a. a high-pass biquadratic filter (e.g., with a frequency cutoff 60 Hz), and/or b. classifying at least a fraction of the recorded sounds as bowel sounds ('BS event spotting').

In embodiments, the recorded audio data is processed by applying at least one filter during and/or after recording.

In embodiments audio recordings may be filtered with a high-pass biquadratic filter (e.g., cutoff: 60 Hz) to remove signal offset. In embodiments audio recordings may be split into non-overlapping audio segments, e.g., with a duration $\delta$=10 s.

In embodiments, the artificial intelligence in step b) comprises a neural network comprising a convolutional neural network (CNN) architecture.

In embodiments, one or more audio segments of the recorded audio data may be converted to log Mel-spectrogram using a certain number of frequency bins, e.g., 128 frequency bins.

In embodiments herein an Efficient-U-Net is used for an audio spectrogram input, returning a binary detection mask in the time domain. In embodiments a 1D mask may be obtained by average pooling along the frequency dimension and, e.g., a Softmax function to the model output. In embodiments the Efficient-U-Net classifies each spectrogram time bin $F_k$ as either containing bowel sounds (BS) or non-bowel sounds (NBS). Thus, the spotting temporal resolution corresponds to the audio spectrogram frame length.

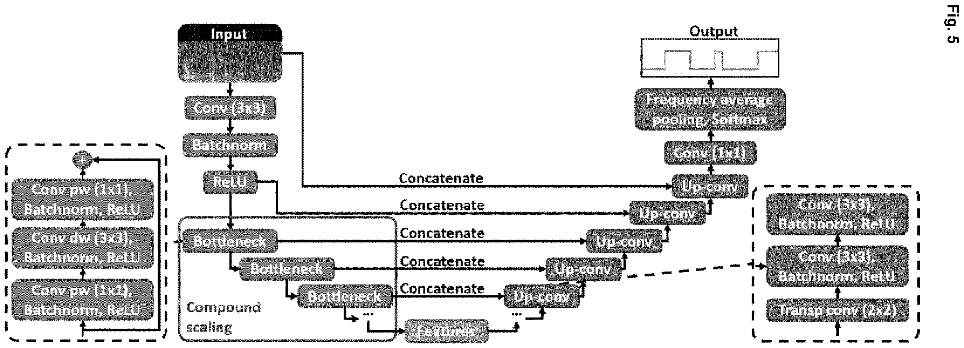

In embodiments an Efficient-U-Net processes audio spectrogram(s) as input and extracts relevant features (e.g., with EfficientNet-B2) during the encoding. In embodiments, features are subsequently decoded, i.e., up-sampled and concatenated with higher resolution features to locate them on the original spectrogram. In embodiments, the obtained 2D features are finally converted to a 'BS detection mask' by applying average pooling along the frequency dimension, and preferably a Softmax operation, to the obtained 1D temporal maps. In embodiments spectrogram frames with highest BS class probability may be identified as containing BS (see, for example, Fig. 5).

Experts may in the context of the invention be, for example, a medical practitioner, a physician, a nurse, a scientist, or a gastroenterologist.

In embodiments, the recorded bowel audio-recording of sounds emitted by the bowel of a subject are converted into a Mel-spectrogram. In embodiments, the conversion into Mel-spectrogram is performed in step b) or c), of the present method, but before the computation of MFCC(-feature)s.

In embodiments, converting recorded bowel sounds into a Mel-spectrogram is performed by a method comprising the steps of

i) filtering the bowel sound signal, preferably by adopting/applying a bandpass Butterworth filter;

ii) conversion of the signal to the frequency domain in order to obtain a frequency spectrum, preferably by performing a Fourier transform on the recorded bowel sound, wherein it is particularly preferred to use a window function, preferably a von Hann or a Hamming window prior to performing the Fourier transform;

iii) passing the frequency spectrum through a Mel-filterbank to obtain a Mel-spectrogram, wherein the Mel-filterbank is preferably composed of T triangular band pass filters, and a logarithm of the filtered frequency spectrum is taken to obtain a logarithmic energy spectrum.

In embodiments, the Mel-spectrogram is subsequently transformed using one or more basis functions in order to obtain the at least one MFCC (-feature).

In embodiments, in step c) computing at least one MFCC(-feature) is performed by/comprises converting the recorded bowel sounds to a Mel-spectrogram and transforming the Mel-spectrogram using one or more basis functions in order to obtain the at least one MFCC. In embodiments the one or more basis functions for transforming the Mel-spectrogram are cosine functions.

In embodiments, in step c) (computing at least one Mel Frequency Cepstral Coefficient (MFCC)-feature from at least a fraction of the recorded bowel sounds extracted before) the one or more basis functions for transforming the Mel-spectrogram are cosine functions.

In particular the present examples demonstrate that by computing Mel Frequency Cepstral Coefficient (MFCC)-features from recorded bowel sounds of a subject and using an artificial intelligence (AI) which takes into account at least one statistical parameter computed from said MFCC-features, a likelihood the subject suffering from a bowel disease or not can be determined with high confidence.

In embodiments, a feature extraction is performed for at least a fraction of the annotated or classified bowel sounds (bowel sound events).

In embodiments, the MFCC feature extraction (according to step c)) comprises the application of type 2 discrete cosine transform to the Mel-spectrogram to obtain Mel-Frequency Cepstrum Coefficients (MFCCs).

In embodiments a certain number of MFCCs (MFCC-features) are selected. In embodiments 1, 2, 3, 4, 5, 6, 7, 8, 9, 10, 15, 20, 25, 30, 35, 40, 45, 50, 55, 60 MFCCs (MFCC-features) are selected.

The present examples show that in some embodiments the advantageous feature set for the screening test may comprise 5, 7, 10 MFCCs or more (mean values).

As shown in the present examples, some of the MFCCs could in embodiments be complemented, or even omitted and re-placed with alternative spectral/perceptual features without affecting the model performance. However, including in embodiments at least one MFCCs, preferably 5 MFCCs, 7 MFCCs, 10 MFCCs or more consistently demonstrated improved results over an analysis using AI, which did not include computing at least one MFCC (e.g., as computational approaches of the prior art). As shown in Table 1 using 17 features including the features skewness of spectral centroid, spectral flatness, strong peak ratio, spectral spread, max magnitude frequency, spectral complexity, zero crossing rate, 10 spectral sub-band energy features, but no MFCCs only yielded a Mean ROC AUC (cross-validation experiments) of 0.68. On the contrary using at least 5 MFCCs (possibly accompanied by further features such as mean energy or a BS location) consistently yielded a Mean ROC AUC (cross-validation experiments) of 0,83. Using more than 20 MFCCs even increased the Mean ROC AUC to 0.85 or more.

The present examples thus demonstrate, how MFCCs features may be advantageously used in the context of the present invention to predict bowel diseases, wherein preferably a one-channel analysis is performed, and/or an analysis over a statistical window (e.g., 10 min of audio recording).

In embodiments, computing of at least one MFCC-feature from bowel sounds in step c) and/or determining at least one statistical parameter in step d) is performed using an artificial intelligence (or machine learning (model)).

In embodiments the processing/analysis steps of identified bowel sounds are not performed by machine learning methods or AI, but analyzed by applying analytical equations, such as, for example, one or more equations disclosed herein. In embodiments computing of at least one MFCC-feature from bowel sounds in step c) and/or determining at least one statistical parameter in step d) is performed by applying one or more analytical equations and/or mathematical calculations. In embodiments at least one MFCC-feature is computed (from bowel sounds) according to/by applying analytical equations and/or mathematical calculations.

In embodiments, a non-limiting list of bowel sound features that may be applied in addition to at least one MFCC(-feature) in the context of the present method comprises:

### A. Spectral Features

i. Mel Frequency Cepstral Coefficients (MFCCs) (may be defined as signal frequency bands representation according to the mel-scale, i.e. human auditory system. In the examples herein, 11 coefficients were employed for the classification). Mel Frequency Cepstral Coefficients (MFCCs) features are the preferred features analyzed/considered in the present method.

ii. Spectral centroid (may be defined as the center of mass of a signal spectrum. Perceptually, the spectral centroid is related to sound brightness).

iii. Spectral complexity (may be defined as a measurement of how much information is needed to estimate the signal spectrum, it is based on the amount of spectrum peaks).

iv. Spectral flatness (may be defined as how much a signal spectrum is noise-like, e.g. white noise has value 1, or similar to a pure tone sound, which has value 0).

v. Spectral spread/spectral centroid (may define the signal spectrum variance around the spectral centroid).

vi. Spectral peak ratio/Strong peak ratio (may be defined as the ratio between the maximum peak's magnitude and the peak band- width in a spectrum. The higher the ratio, the more pronounced is the peak. In embodiments herein the ratio is estimated according to Gouyon and Herrera [44].)

vii. Maximum magnitude frequency/frequency with largest spectrum magnitude (may be defined to measures the frequency having the largest magnitude in a spectrum).

### B. Temporal Features

i. Signal energy (the energy of a signal is defined using as the area under the square of the signal magnitude).

ii. Zero crossing rate (may be defined as the number of time the signal change from positive to negative and vice-versa per unit time).

### C. Perceptual Features

i. Dissonance / sound sensory dissonance (may be defined as the perceptual roughness of an audio signal based on the spectrum peaks. The dissonance can in embodiments herein be estimated according to Plomp and Levelt [45]).

ii. (Signal) Inharmonicity (may be defined as the how much harmonic a signal is based on the signal fundamental frequency and other spectrum peaks).

iii. Signal pitch/sound pitch (may be defined as the signal fundamental frequency. The Yin algorithm [46] may in embodiments herein be used for the estimation).

### D. Other Features

i. BS location (may be defined as the location of the sensor that recorded the bowel sound (BS)).

ii. Sound duration/BS per unit time (in embodiments herein number of BS events occurring within a classification window, i.e. portion of audio recording of duration $\delta$).

iii. Sound-to-Sound (SS) interval (may be defined as the time interval in seconds occurring between consecutive bowel sound (BS) events within a classification window).

In embodiments, in addition to the at least one MFCC-feature computed in step c) further features selected from sound duration, spectral centroid, spectral flatness, signal energy, sound pitch, spectral peak ratio, sound sensory dissonance, signal inharmonicity, frequency with largest spectrum magnitude, spectral complexity and zero crossing rate are computed for at least a fraction of the recorded bowel sounds extracted in b).

The examples herein show that in embodiment it can be advantageous to combine MFCC features with other features, achieving even better prediction results (see e.g., higher ROC-AUC values). However, in embodiment it may be preferred to consider/use more MFCC features in a analysis/calculation that 'other' non-MFCC features. In such embodiments an analysis may comprise/consider, for example, 2, 3, 4, 5 or more additional features, but preferably less than 40, 30, 20 or 10.

In embodiments, the feature extraction comprises the application of a band-pass 8th order Butterworth filter (e.g., passband 60-5000 Hz).

In embodiments, the feature extraction comprises the padding of annotated or classified bowel sounds. In embodiments, an annotated or classified bowel sound is padded, if the bowel sound duration is < 32 milliseconds (ms) or not a multiple of 32 ms, preferably before onset and/or after offset, and preferably with audio data recorded before and/or after the padded bowel sound until the new padded bowel sound duration is equal to 32 ms or a multiple of 32 ms.

In embodiments, the feature extraction comprises power spectrogram calculation of bowel sounds, preferably using a sliding window approach. In embodiments, a sliding window has a specific stride length. In embodiments, the sliding window has length of 32 ms and an 8 ms stride.

In embodiments a sliding window approach is used for/during the classification of bowel sounds. In embodiments a sliding window approach is used for/during feature extraction.

In embodiments, a sliding window may have a length of 10, 16, 25, 32, 64, 128 ms and/or a 1, 2, 3, 4, 5, 6, 7, 8, 9, 10, 16, 30, 32, 60, 64, 128 ms stride.

In some embodiments, for/during the feature extraction the sliding window has preferably a length of 32 ms and a 8 ms stride.

In some embodiments, for/during the classification of bowel sounds (BS spotting) the sliding window has preferably a length of 25 ms and a 10 ms stride.

In embodiments, the sliding window corresponds to the spotting temporal resolution of the used CNN, e.g., an Efficient-U-Net.

In embodiments, subsequently a Hanning windowing may be applied to the sliding windows. In embodiments, Hann windowing is used for the spectrogram calculation. In embodiments every sliding window is multiplied by Hann window before calculating spectrogram.

In embodiments, a Hanning window is applied for/during the classification of bowel sounds (BS spotting).

In embodiments, a Hann window is applied for/during MFCC feature extraction.

In embodiments, the feature extraction comprises application of a Mel-filterbank to the spectrogram, to obtain a Mel-spectrogram.

In embodiments, the application of a Mel-filterbank comprises the conversion of a frequency from Hz to Mel scale according to following equation:

$$m = 2595 \log_{10}\left(1 + \frac{f}{700}\right),$$

where f is the frequency in Hz and m is the frequency in mels.

In embodiments, the application of a Mel-filterbank comprises the division of the frequency into discrete, overlapping bins according to Mel-filterbanks frequency bands.

In embodiments, the application of a Mel-filterbank comprises the multiplication of each Mel-filterbank (triangular window) by the corresponding power spectrogram frequency bin and sum the power spectrogram of all frequencies included in the bin.

In embodiments, a Mel-filterbank is applied to the spectrogram to obtain a Mel-spectrogram comprising the steps of:

i. Converting frequency from Hz to Mel scale according to following equation:

$$m = 2595 \log_{10}\left(1 + \frac{f}{700}\right)$$

,Where $f$ is the frequency in Hz and $m$ is the frequency in mels,

ii. Dividing frequency into discrete, overlapping bins according to Mel-filterbanks frequency bands, and

iii. Multiplying each Mel-filterbank (triangular window) by the corresponding power spectrogram frequency bin and sum the power spectrogram of all frequencies included in the bin.

In embodiments, the feature extraction comprises the conversion of the Mel-spectrogram amplitude to decibel (dB) scale.

In embodiments in step c) at least 5 MFCC(-feature)s, preferably at least 10, are computed.

In embodiments, the feature extraction comprises the averaging of MFCCs across all or a fraction of sliding windows used to calculate BS Mel-spectrogram, e.g., across all 32-ms sliding windows, obtaining thus a certain number of MFCCs per bowel sound, e.g., 20 MFCCs per bowel sound (event).

One advantage of the present method is that the present method can achieve a high prediction performance, such as a high prediction reliability and reproducibility, through the usage of MFCCs. The use of MFCCs has not been suggested or described before in the art for the analysis of bowel sounds and the prediction of a bowel disease and as the examples show (e.g., Example 2, Table 1) a reliable prediction may already be achieved using 5 or more MFCCs. Further, particularly good results can be achieved, for example, with a group of features comprising more than 5 MFCCs (MFCC features), more than 10 MFCCs. With 20 MFCCs a particularly precise prediction may be achieved (ROC-AUC of 0.85). However, the examples (e.g., Example 2, Table 1) show, that if no MFCCs are used, the prediction performance is significant lower than with the usage of MFCCs.

In embodiments, after feature extraction, the continuous recording may be divided into classification time windows. In embodiments said classification windows may have a length of preferably around 10 minutes or of 1, 2, 3, 4, 5, 6, 7, 8, 9, 10, 15, 20, 25 or 30 minutes.

In contrast to prior art methods, e.g. of WO 2019/157552 A1 (Marshall et al.), the present method is able to reliably predict the likelihood of a bowel disease in a subject from only a few minutes of audio recording, e.g., from only 10 minutes. In embodiments the precision of the present method, however, may be even more enhanced by using audio recording extending beyond the duration of 10 minutes.

However, the present method enables an analysis of BS features detected in any subject, either suffering from different disease conditions or a healthy subject. In embodiments of the present method an AI (e.g., GBC) may be trained to detect one or more audio classification time windows of preferably only 10 min in an audio recording, such that subjects suffering from a bowel disease can successfully be identified with a mean AU-ROC above 0.83, regardless of the annotation tool employed to annotate BS in the audio recordings (computational-based or by an expert).

In embodiments, the at least one classification time window has a length of between 1 and 60 Minutes, preferably of about 10 Minutes.

In embodiments, subsequently for each classification time window, feature statistics (mean of MFCCs) of bowel sounds (bowel sound events) occurring within the classification time window are calculated. In some embodiments feature statistics of bowel sounds comprise mean and variance of MFCCs.

In embodiments, at least a fraction or each classification window may be classified (e.g., in embodiments with Gradient Boosting Classifier) as either being recorded from a subject suffering or suspected of suffering from a bowel disease or a healthy subject.

In embodiments the prediction in step e) comprises a Bayesian approach, wherein the likelihood is preferably the probability of a bowel disease in the subject.

In embodiments, the artificial intelligence (AI) employs a Gradient Boosting Classifier (GBC) in step e).

In embodiments, the audio recording within the at least one classification time window is split into audio segments of between 1 and 60 seconds, preferably of about 10 seconds.

In embodiments, the duration of the audio recording in step a) exceeds the duration of the at least one classification time window, wherein the classification time window is a sliding classification time window, and wherein in step c) in the sliding classification time window at least one MFCC-feature is computed from at least a fraction of the recorded bowel sounds.

In embodiments, statistical analysis is applied to one or more classification time windows for the prediction according to step e).

In embodiments, the audio recording in step a) is has a duration of over 10 minutes, and wherein the sliding classification time window has a length of 10 minutes.

In embodiments, the artificial intelligence employed in step b) has been trained on manually annotated bowel sound data comprising a Leave-One-Participant-Out (LOPO) cross-validation.

In embodiments for each classification time window, bowel sound (BS) features mean and, if applicable, variance (or standard deviation) may be calculated from the detected BS events within the window.

In embodiments, the at least one filter comprises a band-pass filter and/or a recursive linear filter, preferably a high-pass biquadratic filter, more preferably a high-pass biquadratic filter applying a cutoff of 60 Hz.

In embodiments, the audio-recording in step a) is recorded during one or more different digestive phases of the subject.

In embodiments, the one or more digestive phases comprise one or more phases of sedentary activities, physical exercise, food intake and/or digestion.

One advantage of the present method is the robustness against acoustic noise, e.g., due to arbitrary room sounds, other body sounds (e.g., breathing, voice), and sounds related to physical activities (e.g., cloth rubbing). The method could be implemented in a form of (a) everyday, continuous monitoring, (b) time-limited, momentary screening; and in locations at home, on the move/mobile, or in specific environments, including laboratories, acoustic chambers, etc.

In embodiments, the audio-recording in step a) is recorded (or provided) using a wearable recording system (e.g., a smart garment or smart shirt or smart vest) comprising one or more microphone(s).

In embodiments, one or more microphone(s) (e.g., of a wearable recording system) are placed on the stomach and/or the small intestine and/or the large intestine of the subject for audio-recording.

In embodiments, one or more microphone(s), preferably two or more microphones, are used to provide (or record) an audio-recording in step a). In embodiments two or more microphones are used to provide an audio-recording in step a), wherein the audio data recorded by the two or more microphones is fused (combined) for (in advance of) audio data analysis according to (one or more of) steps b)-e). In embodiments the audio data recorded by two or more microphones is fused (combined) for (in advance of) bowel sound extraction in step b) and/or for predicting the likelihood of a bowel disease in step e). In embodiments audio data (sounds) recorded from the bowel of a subject and/or provided in step a), which has been recorded by more than one microphone and/or other sources is fused or combined prior to: audio data analysis according to steps b)-e), bowel sound extraction in step b) and/or for predicting the likelihood of a bowel disease in step e). In embodiments audio data recorded by two or more microphone(s) is combined for (prior to) audio data analysis according to steps b)-e), bowel sound extraction in step b) and/or for predicting the likelihood of a bowel disease in step e).

In embodiments, the one or more microphone(s) may be constructed according to different principles (e.g., piezoelectric, piezoresistive, capacitive, and/or inductive) such that bowel sounds may be captured in embodiments either as airborne particle waves and/or tissue vibration. In embodiments one or more microphones may be constructed as a micro-electromechanical systems (MEMS) transducer.

In embodiments analyzing BS at different abdominal locations could improve the IBD detection performance even more, as abnormal BS patterns originated by the inflamed region are more likely to be observed. The BS feature selection used in the examples herein showed that BS location may in embodiments be relevant to predict a bowel disease even more reliably.

In one specific embodiments, the present method comprises the steps of:

I. Recording/providing a continuous audio recording (using one or more microphones) of the abdomen of a subject,

II. Subjecting the recording to audio preprocessing comprising

i. Applying a high-pass biquadratic filter, and/or

ii. Classifying at least a fraction of the recorded sounds as bowel sounds ('BS event spotting'), preferably either manually (e.g., by an expert) or with an AI model (e.g., an Efficient-U-Net).

III. Extracting one or more features for at least a fraction of the annotated or classified bowel sounds (bowel sound events), comprising

i. Applying a band-pass 8th order Butterworth filter,

ii. If the duration of a bowel sound is < 32 ms or not a multiple of 32 ms, padding each or a fraction of the bowel sounds before onset and/or after offset with audio data recorded before and/or after a respective bowel sound until the bowel sound duration is equal to 32 ms or a multiple of 32 ms,

iii. Calculating a power spectrogram of each or a fraction of the bowel sounds using a sliding window, e.g., of 32 ms with 8 ms stride, wherein preferably Hann windowing is used for the spectrogram calculation, wherein preferably every sliding window is multiplied by Hann window before calculating a spectrogram,

iv. Applying a Mel-filterbank to the spectrogram to obtain Mel-spectrogram, comprising

i. Converting the frequency from Hz to Mel scale according to the equation:

$$m = 2595 \log_{10}\left(1 + \frac{f}{700}\right),$$

wherein $f$ is the frequency in Hz and $m$ is the frequency in mels,

ii. Dividing a frequency into discrete, overlapping bins according to Mel-filterbanks frequency bands,

iii. Multiplying each Mel-filterbank (triangular window) by the corresponding power spectrogram frequency bin and sum the power spectrogram of all frequencies included in the bin,

v. Converting the Mel-spectrogram amplitude to decibel (dB) scale,

vi. Applying type 2 discrete cosine transform to the Mel-spectrogram to obtain Mel-Frequency Cepstrum Coefficients (MFCCs), and selecting a certain number of MFCCs,

vii. Averaging MFCCs across all sliding windows used to calculate the bowel sound Mel-spectrogram, obtaining thus MFCCs per bowel sound (event).

IV. Divide continuous recording into classification windows of 10 minutes. For each 10-minute classification window, calculate feature statistics (mean and variance of MFCCs) of BS events occurring within the 10-minute window.

V. Classify (with Gradient Boosting Classifier) each 10-minute classification window as either being recorded from an IBD patient or a healthy participant.

In one aspect the present invention relates to the use of the method according to the invention for assisting the diagnosis of a bowel disease in a subject. In another aspect the present invention relates to the use of the method according to the invention for predicting the presence of a bowel disease in a subject and/or the likelihood thereof.

In another aspect the present method relates to the use of the method according to the invention for predicting the likelihood of a bowel disease in a subject.

In embodiments, the likelihood may be expressed as a numerical ((bowel disease) prediction) score, wherein 0 indicates the lowest possible likelihood of a bowel disease (exclusion) and 1 indicates the highest likelihood of a bowel disease. In embodiments, a high likelihood over 50% or over 0.5 may be indicative of the presence of a bowel disease in the subject and may indicate to a physician or medical professional to prescribe further diagnostic tests (e.g., more invasive tests) to confirm the presence of a bowel disease in the subject. Subsequent diagnostic test may be biopsies and/or colonoscopy.

The present examples show that the present method is able to determine a bowel disease probability of > 0.50 for all patients, regardless of the disease condition. In particular, IBD was predicted by GBC with accuracy 0.91. The results show that this method could be potentially employed in clinical practice as a gastrointestinal disease, such as IBD or IBS, screening test prior to use or instead of more invasive investigations such as endoscopy.

In embodiments, the present method may be used for the prognosis, diagnosis, treatment guidance and/or monitoring, risk stratification and/or risk assessment of a subject diagnosed or suspected to suffer from a bowel disease.

In embodiments, the present method may be used for the prognosis, diagnosis, treatment guidance and/or monitoring, risk stratification and/or risk assessment of a bowel disease in a subject.

In another aspect the present method relates to a computer-implemented method comprising the steps of c)-e) of the method according to the invention. In embodiments the present method relates to a computer-implemented method comprising the steps of b)-e) of the method according to the invention. In embodiments where step b) comprises the annotation (or classification/identification) of bowel sounds using an artificial intelligence (AI, or machine learning (model)) said step may be computer-implemented as well.

In another aspect the present method relates to a software or computer program for predicting the likelihood of a bowel disease in a subject, wherein said software or computer program product comprises commands to perform computational steps c)-e) of the method according to the present invention.

In embodiments the present method relates to a software or computer program for predicting the likelihood of a bowel disease in a subject, wherein said software or computer program product comprises commands to perform computational steps b)-e) of the method according to the present invention. In embodiments step b) may be considered to be a computational step, e.g., if the annotation (or classification/identification) of bowel sounds in step b) is performed using an artificial intelligence (AI, or machine learning (model)).

In embodiments the present computer-implemented method or the present computer program may be used for assisting the diagnosis of a bowel disease in a subject. In embodiments the present computer-implemented method or the present computer program may be used for predicting the presence of a bowel disease in a subject and/or the likelihood thereof.

In embodiments the present computer-implemented method or the present computer program may be used for predicting the likelihood of a bowel disease in a subject.

In embodiments, a score, likelihood, or prediction result determined by the present method may be displayed to a user, e.g., a medical professional, via a graphical user interphase (GUI), optionally comprising further instructions/suggestions regarding the next steps to be performed, e.g., potential subsequent examinations and/or tests, based on the prediction made by the present method.

Particular aspects of the present invention may be computer-implemented. Accordingly in embodiments the present method may be a computer-implemented method. The person skilled in the art is aware of which aspects and features of the present invention may be computer-implemented.

In another aspect the present method relates to a computer-readable storage device, comprising a software or computer program product according to the present invention.

Each optional or preferred feature of the invention that is disclosed or described in the context of one aspect of the invention is herewith also disclosed in the context of the other aspects of the invention described herein.

All features disclosed in the context of the method according to the invention also relate to and are herewith disclosed also in the context of the computer implemented method, the computer program, storage device or any use described herein, and vice versa.

The various aspects of the invention are unified by, benefit from, are based on and/or are linked by the common and surprising finding of the unexpected advantageous effects of the present method for assisting the diagnosis of a bowel disease by identifying and characterizing bowel sounds of a subject into bowel sounds indicative of a bowel disease or a healthy subject.

## DETAILED DESCRIPTION OF THE INVENTION

All cited documents of the patent and non-patent literature are hereby incorporated by reference in their entirety.

The present invention is directed to a method for assisting the diagnosis of a bowel disease from audio-recordings of sounds emitted by the bowel of a subject by using an artificial intelligence (AI) to classify the emitted bowel sounds as being indicative for the individual suffering from a bowel disease or for a healthy individual. The invention further relates to the use of the present method for the diagnosis of a bowel disease and to a computer-implemented method and software or computer program for predicting the likelihood of a bowel disease in a subject.

Herein a "subject" or "patient" may be a vertebrate, preferably a mammal, more preferably a human subject or patient. In the context of the present invention, the term "subject" or "patient" includes both humans and animals, particularly mammals, more particularly humans.

Inflammatory bowel disease (IBD) describes group of inflammatory disorders involving longstanding (chronic) inflammation of tissues in the digestive tract, e.g., the colon and small intestine. IBD comprises Crohn's disease and ulcerative colitis (UC) as the major prevalent types.

Irritable bowel syndrome (IBS) is a chronic condition affecting the gastrointestinal tract comprising the intestines and stomach. IBS symptoms comprise cramping, abdominal pain, bloating, gas, and diarrhea or constipation, or both.

The Mel Frequency Cepstral Coefficients (MFCC) are presently often used for automatic speech recognition, as they have the advantage of leading to a compact representation of the frequency spectrum. The Mel in the name describes the perceived pitch. Mathematically formulated, the impulse response of the filter is convoluted with the excitation signal to produce the speech signal. When calculating the Cepstrum, the convolution operation is transformed into an addition based on the logarithm, which is easy to separate, allowing the speech signal to be separated into excitation and source. In some embodiments MFCCs may be calculated by the steps of: i) Dividing the input signal into blocks or windows that can be multiplied by e.g., Hamming window function to avoid edge effects (such that Hamming window(s) is/are multiplied by the windowed signal). Overlapping windows are common. ii) (Discrete) Fourier transform of each window, iii) Generation of the magnitude spectrum. iv) Conversion of the spectrum frequency scale to Mel-scale. v) Mapping the frequency bins to the Mel-filterbanks by applying triangular filters. vi) Final decorrelation by either a Discrete Cosine Transform or a Principal Component Analysis. vii) The MFCCs are the amplitudes of the resulting cepstrum [49-52].

While it is known that MFCCs may be used for automatic speech recognition, as they have the advantage of leading to a compact representation of the frequency spectrum, it was a surprising finding of the inventors that computing MFCCs and using these features as an input for an AI model may yield a substantially improved performance on predicting whether a corresponding audio recording would stem from an individual suffering of IBD or not.

A Fourier transform is a mathematical technique for converting a time function into one expressed in terms of frequency or, in other words, that decomposes aperiodic signals into a continuous spectrum. The Discrete Fourier Transform (DFT) is a transform from the field of Fourier analysis that maps a discrete-time finite signal, which is periodically continued, to a discrete, periodic frequency spectrum. In digital signal processing, the function is any quantity or signal that varies over time, such as the pressure of a sound wave sampled over a finite time interval (often defined by a window function).

In Fourier analysis, the cepstrum is the result of computing the inverse Fourier transform (IFT) of the logarithm of the estimated signal spectrum. The method is a tool for investigating periodic structures in frequency spectra. In general, a cepstrum is the result of the following calculation sequence: a) transforming a signal from the time domain to the frequency domain, b) computation of the logarithm of the spectral amplitude, c) transformation to frequency domain, in which the independent variable again represents a time scale. Mathematically it is concerned with the problem of deconvolution of signals in the frequency space. In general, the cepstrum resembles a concentration of the energy of the periodic frequency structure component on the original spectrum, and it is considered to give more weight to the low-amplitude component and less weight to the high-amplitude component in the logarithmic conversion of power, thus emphasizing the small signal period. One advantage of cepstrum analysis is that it allows the decomposition of a signal into individual periodic frequency components and thus allows to investigate periodic structures in frequency spectra. In sound processing, the Mel Frequency Cepstral Transform can be used to calculate the short-term power spectrum of a sound in the form of Cepstral coefficients.

In general, a 'Butterworth filter' is a type of signal processing filter designed to have a frequency response that is as flat as possible in the passband.

Herein a 'classification time window' is considered a fraction or part of an audio recording of a certain/defined duration (time), which may be used for downstream analysis.

In a 'sliding window' analysis or 'sliding window' approach (short 'sliding window') test statistics are plotted with a sliding window at a certain length along a (certain stream or sequence of) data. In other words, in a sliding window approach, a window of specified length, moves (slides) over the data, whereby the statistic is computed over the data in the window. Herein, for example, in embodiments a sliding window approach is applied in the context of a sliding classification time window.

In embodiments, the recording is subjected to audio preprocessing. In embodiments said audio preprocessing may comprise applying a. a high-pass biquadratic filter (e.g., with a frequency cutoff 60 Hz), and/or b. classifying at least a fraction of the recorded sounds as bowel sounds ('BS event spotting').

In embodiments audio recordings may be filtered with a high-pass biquadratic filter (e.g., cutoff: 60 Hz) to remove signal offset. In embodiments audio recordings may be split into non-overlapping audio segments , e.g., with a duration $\delta$=10 s. In embodiments each audio segment $S_i$ is defined as a set of samples:

$$(1) \qquad S_i = \{t_c : i \cdot \delta \leq t_c < (i+1) \cdot \delta, i \in N\},$$

where $t_c = n \cdot \dfrac{1}{fs}$ , $n \in N$ are timeseries samples.

In embodiments, for every $S_i$, the audio spectrogram time bins $F_k$ may be defined as:

$$(2) \qquad F_k = \{t_c : k \cdot \sigma \leq t_c < k \cdot \sigma + \gamma, k \in S_i\},$$

where $t_c = n \cdot \dfrac{1}{fs}$, $n \in N$ are timeseries samples and sliding window duration $\gamma = 25\,ms \cdot f_s$ and stride length $\sigma = 10\,ms \cdot fs$, all in timeseries samples.

In embodiments, an annotated BS event $e_j$ can be denoted as a set of timeseries samples as follow:

$$(3) \qquad e_j = \{t_{j,s}, \ldots, t_{j,e}, j \in \mathbb{N}\},$$

where $t_{j,s}$ and $t_{j,e}$ are the onset and offset of BS event $e_j$ in timeseries samples.

In embodiments, for model learning, BS manual annotations may be converted to audio spectrogram ground truth masks according to the approach of Ficek et al. 2021 [48]:

Therein in embodiments a spectrogram frame $F_k$ may be defined as containing BS ($F_{k,BS}$), if the time overlap $\varepsilon$ between the spectrogram frame and a BS event $e_j$ was $\geq$ 50%. Thus, for set $F_{k,BS}$:

$$(4) \qquad F_{k,BS} := F_k : \frac{|F_k \cap e_j|}{|F_k \cup e_j|} \geq \epsilon,$$

where $\varepsilon$ = 0.5 is the temporal overlap and $|\cdot|$ is the set cardinality. Otherwise, the spectrogram frame may be denoted as containing NBS ($F_{k,NBS}$), i.e. NULL class. In embodiments, the supersets of all spectrogram frames may be defined as $F_{k,BS} \in F_{BS}$ and $F_{k,NBS} \in F_{NBS}$. Therefore, for each audio segment $S_i$, preferably from EffUNet a binary mask $M_i$ denoted as $F_k$ is obtained. As in embodiment with the log-Mel spectrograms the binary masks $M_i$ may be zero-padded along the time axis to obtain 1$\times$1056 time bin masks.

In embodiments, an EffUNet encoder, i.e. EfficientNet-B2, may be initialised with pretraining weights from AudioSet. In embodiments pretraining on a large variety of sounds could improve the spotting robustness against background noise and other artefacts. In embodiments the decoder weights may be initialised with He initialisation [40].

In embodiments, for training and data augmentation EffUNet training parameters may be selected according to the PSLA pipeline [38]. In embodiments, after model initialisation, EffUNet may be trained for, e.g., 25 epochs, using, e.g., an imbalanced batch size of 3.

In embodiments, an initial learning rate of = 1 . $10^{-4}$ may be subsequently reduced starting from the $6^{th}$ epoch with a decay of 0.85 for each epoch.

In embodiments, Adam optimiser [41] may be used with weight decay of 5 $\cdot$ $10^{-7}$, $\beta_1$ = 0.95, $\beta_2$ = 0.999.

In embodiments, the following loss function may be used for optimisation:

$$(5) \qquad L(y, \hat{y}) = L_1(y, \hat{y}) + L_2(y, \hat{y}),$$

where $L_1(y,\hat{y})$ is the cross-entropy loss calculated between the prediction $\hat{y}$ and the ground truth $y$, and $L_2(y,\hat{y})$ is the dice loss.

In embodiments, during the training, the input audio spectrograms may be randomly transformed to improve the model generalisation. In embodiments, on each batch, time-frequency masking may be applied to up to 24 frequency bins and up to 10% of the time bins. In embodiments, spectrograms may be randomly shifted along the time axis with a maximum shift of $\pm$10 time bins. Random white noise may probably added to the input, too. For the evaluation, in embodiments the model weights obtained at the end of the training may be selected.

In embodiments, for spotting implementation the binary masks $M_i$ obtained from EffUNet may be converted to onset/offset predictions of BS events. Spotted BS events $\hat{e}_J$ were described as:

$$(6) \qquad \hat{e}_j = \{t_c : t_c \in D_{i,BS}\},$$

$$(7) \qquad D_{i,BS} = \{\hat{F}_{i,BS} : i \leq j < i + N; i, j \in N\},$$

where $t_c = n \cdot \frac{1}{fs}$ , $n \in N$ are timeseries samples, and $D_{i,BS}$ is the set of N consecutive overlapping audio spectrogram time bins $\hat{F}_{i,BS}$ that were detected as containing BS.

The term "machine learning (ML)" refers to a field directed to methods that "learn," i.e., methods that use data to improve performance on a variety of tasks. Machine learning is commonly considered part of artificial intelligence (AI). A machine learning algorithm is able to construct a model based on sample data (training data). Machine learning models or AI are constructed and subsequently trained to make independent (without being programmed to) decisions or predictions.

A "feature" in the field of machine learning or AI refers to a single measurable attribute or characteristic of a data occurrence. Feature selection is a crucial element of algorithms in pattern recognition, and classification.

In general, a neural network (NN) comprises a sequence of mathematical operations that processes the input into the output, organized in layers. A "neural network" in the context of machine learning refers to a specific type of machine learning in which inputs are converted by a network of artificial neurons, or 'nodes', organized in a series of layers. In artificial neural networks, the connections of the biological neuron are preferably modelled as weights between nodes. In an artificial network the signals received by a neuron are preferably summed and processed by an activation function.

The term "deep learning" is a specific type of machine learning methods and relies on artificial neural networks called "deep" neural networks. A deep neural network (DNN) consist of multiple layers through which data is transformed.

An UNet is a Convolutional Neural Network (CNN) that was originally proposed for biomedical image segmentation (Ronneberger et al., 2015. Lecture Notes in Computer Science vol 9351; 2015. p. 234-241). The model name is given by its U-shaped architecture, which is composed by an encoder followed by a decoder network. The encoder extracts relevant features from the DNN input, and the decoder generates a segmentation mask by up-sampling features from the encoder's last layer and concatenating them with higher resolution features extracted from the encoder's earlier layers. Each block of the decoder is therefore preferably composed by a $2 \times 2$ transposed convolution (up-conv), preferably followed by two $3 \times 3$ convolutions. Up-sampling restores the original input resolution, and the concatenation improves localisation of the extracted features. A final convolutional layer classifies each input point, i.e., in embodiments, each time-frequency bin of audio spectrogram $F_k$ with k e N.

Herein an Efficient-U-Net (EffUNet) DNN model architecture is preferably based on UNet and EfficientNet models (Tan et al., 2019; doi: 10.48550/arXiv.1905.11946), hence the name Efficient-U-Net (EffUNet).

Herein a 'Gradient Boosting Classifier' (GBC) refers to a classifier of a machine learning technique used e.g., in regression and classification tasks. Gradient boosting (GB) is a machine learning method. GB commonly provides a prediction model in the form of an ensemble of weak prediction models, such as models making very few assumptions about data, which are typically simple decision trees. In simple terms, gradient boosting is considered an ensemble method that is based on (simple) decision trees. Herein, in embodiments an AI model may employ a Gradient Boosting Classifier to predict the presence of a bowel disease in a patient.

In embodiments 'taking into account the previously determined statistical parameter of MFCC-features' refers to the analysis and/or interpretation, preferably the analysis by a machine learning (ML) model or AI, of a previously determined statistical parameter (e.g., mean and/or variance) preferably based on previously learned data context (training data) for the final decision making, e.g., regarding the existence of a bowel disease. In other words, preferably the AI or machine learning model interprets the presently processed/analyzed data in view of the data used for training the AI or ML model, from which it has preferably learned to distinguish sounds indicative of a bowel disease from 'normal' sounds of a healthy digestive system.

The term "graphical user interface" or "GUI" generally refers to a form of user interface allowing the interaction of a user with electronic devices. This is facilitated through a graphical representation, graphical icons and/or audio-visual or haptic instead of text-based user interfaces, typed command labels or text navigation. In embodiments, a GUI may be a system of interactive visual components for computer software that displays objects, instructions or data that convey information and represent actions that can be taken by as user.

The term "at least one" may herein refer to at least one, more than one, at least two at least three, at least four, at least five, at least six, at least seven, at least eight, at least nine, at least ten, at least fifteen, at least twenty, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 500, 1000, 10.000.

## FIGURES

The invention is further described by the following figures. These are not intended to limit the scope of the invention but represent preferred embodiments of aspects of the invention provided for greater illustration of the invention described herein.

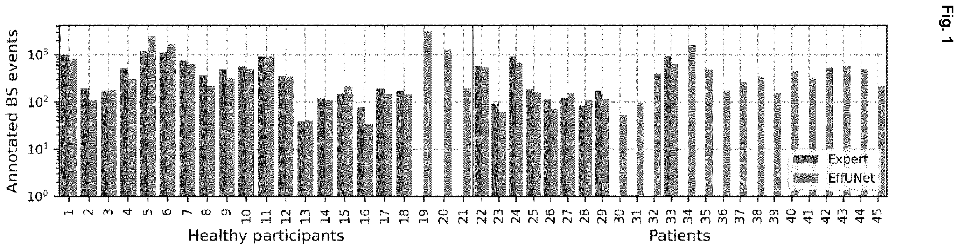

**Figure 1:** Number of annotated BS events per participant. Pairs of raters annotated the recordings from 27 participants, identifying 11482 events. Our spotting model EffUNet detected 22442 BS on the full dataset, comprising 45 participants.

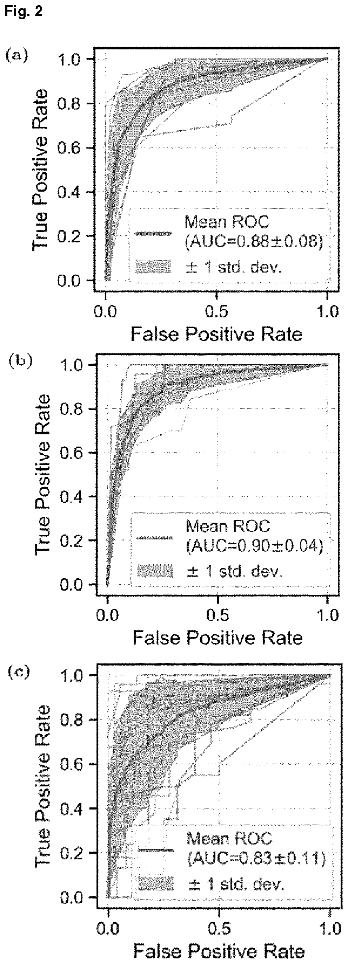

**Figure 2:** (a) Classification ROC curves across all CV folds using expert BS annotations on the study group including 27 participants (9 IBD patients). GBC achieved a mean AUROC of 0.89. (b) Classification ROC curves across all CV folds using EffUNet BS annotations on the study group including 27 participants (9 IBD patients). GBC achieved a mean AUROC of 0.89. (c) Classification ROC curves across all CV folds using EffUNet BS annotations on the full dataset (21 healthy participants and 24 IBD patients). GBC achieved a mean AUROC of 0.83.

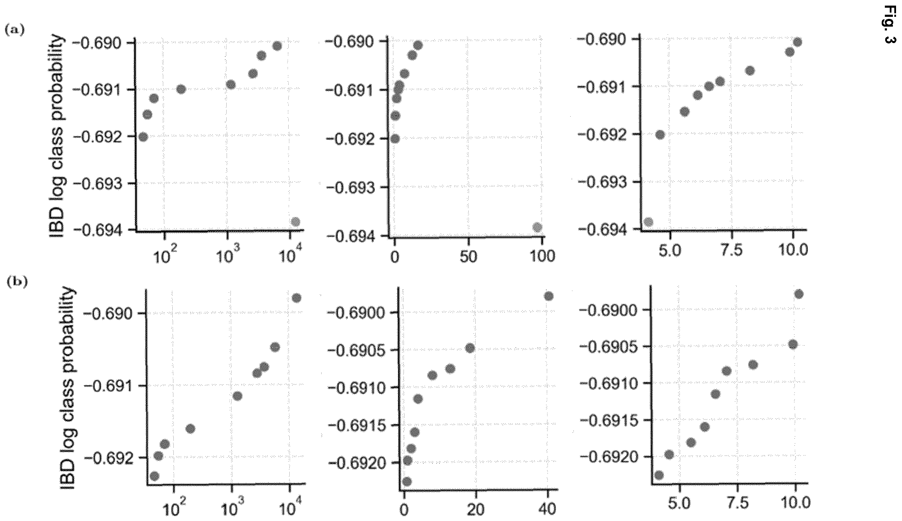

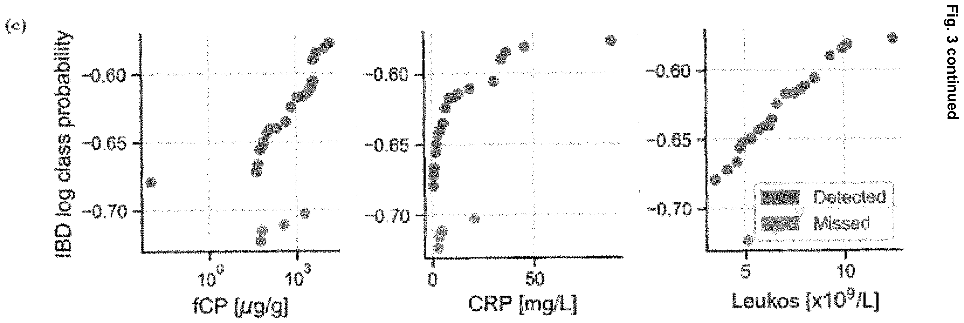

**Figure 3:** Quartile-Quartile (QQ) plots between IBD class probability and inflammation biomarkers collected from the patient group. To assess disease remission, inflammation biomarker ranges vary depending on used test kit, patient ethnicity/age, and clinician experience, rendering thus the evaluation challenging. (a) QQ plot obtained from the expert annotated dataset of 27 participants. (b) QQ plot obtained from the annotated dataset of 27 participants using EffUNet annotations, (c) QQ plot obtained from the full dataset annotated by EffUNet. The data points of the upper curve (dark gray) indicate detected, the lower data points forming a lover curve (light gray) indicate missed.

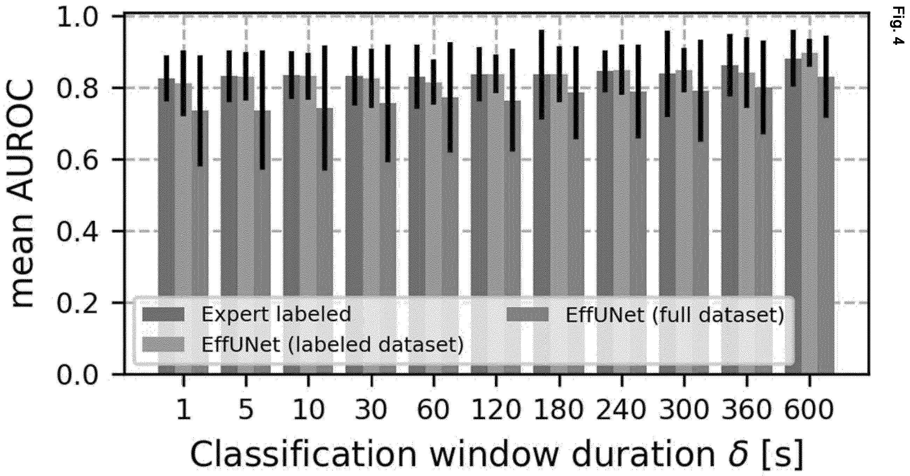

**Figure 4:** Mean AUROC across all datasets over classification window duration. Error bars indicate AUROC standard deviation (std). The bars of the graph on the left side of each data group (each classification window duration of 1, 5, 10, 30 etc. seconds represent a data group) indicate the labelling by an expert, respectively. The bars in the middle of each data group indicate the labelling by EffUNet (labelled dataset; light gray), respectively. The bars in the right side of each data group indicate the labelling by EffUNet (full dataset), respectively.

**Figure 5:** Architecture of the EffUNet model developed for BS spotting. EffUNet took as an input an audio spectrogram and returned a temporal binary mask labeling each spectrogram frame as containing either BS or not. The EffcientNet-B2 encoder (boxes on the left side of scheme) extracts relevant acoustic features that are subsequently up-sampled and located on the spectrogram be the decoder (boxes on the right side of the scheme; after the concatenation) to identify BS occurrences on the time axis. Before the detection, features are averaged along the frequency. Conv: Convolution; dw: depth wise; pw: pointwise, batch norm: batch normalization; transp: transposed.

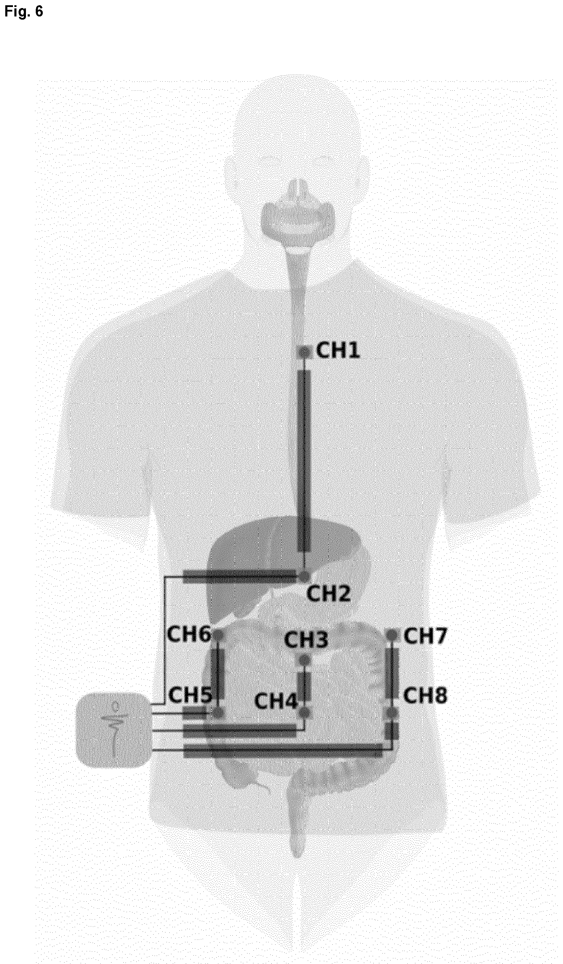

**Figure 6:** GastroDigitalShirt microphone matrix based on the 9-quadrants abdominal map reference (human model adapted from [47], under Creative Commons Attribution 4.0 International License).

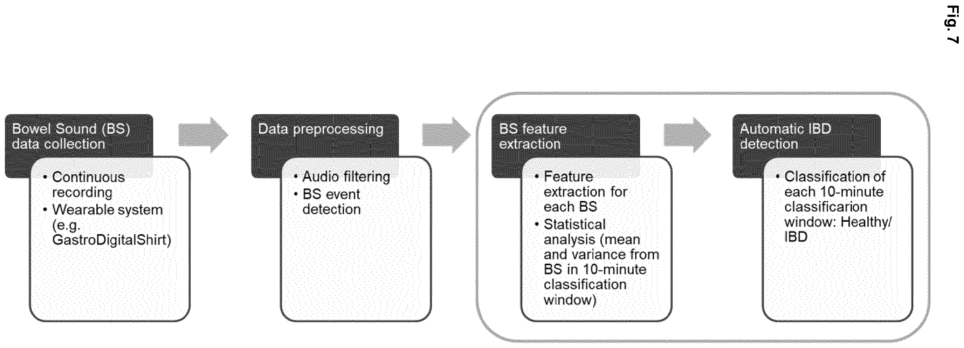

**Figure 7:** Schematic overview depicting the subsequent steps of one embodiments of the present invention.

## EXAMPLES

The invention is further described by the following examples. These are not intended to limit the scope of the invention but represent preferred embodiments of aspects of the invention provided for greater illustration of the invention described herein.

In the present example the inventors investigate Bowel Sounds (BS) to detect patients diagnosed with Inflammatory Bowel Disease (IBD) based on continuous abdominal recordings. BS were recorded from 24 IBD patients with varying disease activity and 21 healthy controls across different digestive phases using a smart T-Shirt with embedded miniaturized microphones. In total, approx. 260 h of audio data were inspected by expert raters and thereof 136 h were manually annotated. Based on the annotated BS, a Deep-learning-based pattern spotting algorithm was trained to retrieve BS instances. Subsequently, BS features were extracted from non-overlapping windows of duration $\delta =$ 10 min and a Gradient Boosting Classifier was trained to recognize patients. Stratified group K-fold cross-validation experiments yielded a mean Area Under the Receiver Operating Characteristic curve (ROC curve) $\geq 0.83$ regardless of manual or spotting-based BS retrieval.

The present results for IBD classification demonstrate that the proposed method may efficiently support IBD diagnosis in a screening test.

In particular the examples demonstrate that by computing Mel Frequency Cepstral Coefficient (MFCC)-features from recorded bowel sounds of a subject and using an artificial intelligence (AI) which takes into account at least one statistical parameter computed from said MFCC-features, a likelihood the subject suffering from a bowel disease or not can be determined with high confidence.

The inventors further explore the link between IBD classification and disease activity.

**Patients, materials and methods**

The following methodologies were used in examples 1, 2, 3 and 4; however, with different/modified feature set used for training the IBD classification model, if indicated accordingly.

Demographics

The clinical study included 21 healthy participants and 24 IBD patients, recruited between March 2020 and November 2021. Among the patients, 14 were diagnosed with Chron's Disease (CD) and 10 with Ulcerative Colitis (UC) and. For each patient, fCP, CRP concentrations, and leukocyte counts from blood and stool samples were analyzed to assess IBD activity. Based on biomarker levels, 14 patients showed disease activity. Table 1 illustrates the population characteristics of the employed dataset.

IBD classification

We used a binary GBC [31] to detect classification windows $S_i$ recorded from IBD patients. On the annotated dataset comprising 18 healthy participants and 9 IBD patients, the model was trained using 50 estimators, exponential loss and a learning rate of 0.0001. On the full dataset, we trained the binary GBC using 200 estimators, exponential loss and a learning rate of 0.001. During the training, Friedman mean squared error score was employed as criterion to evaluate the quality of a split. The classifier was evaluated on the dataset using a stratified group k CV, where k was chosen based on the amount of participants included in the BS annotation. The training set ('trainset') of each k fold was resampled using SMOTE (Synthetic Minority Oversampling Technique) to balance the amount of classification windows extracted from IBD patients and healthy controls (applying resampling on the trainset to minimize data imbalance).

The $S_i$ window predictions were merged for every participant using hard majority voting. Hence, a participant was classified as IBD patient if at least 50% of the classification windows were detected as being recorded from an IBD patient. The participant was classified as healthy otherwise.

Study procedure

The study included healthy participants with no reported gastro-intestinal disorder and patients with diagnosed IBD. Participants had no food intolerance to the meals served during the study. Pregnant or breastfeeding women and UC patients that underwent a total colectomy were excluded from the study. Study sessions began in the morning before breakfast in the lab, after participants provided written consent. BS were recorded continuously for one hour before (fasting phase) and one hour after breakfast (postprandial phase), including meal intake. Participants could interrupt the recordings anytime, e.g. for toilet visits. The GastroDigitalShirt [32], a smart T-Shirt embedding eight miniaturized microphones (Knowles, SPH0645LM4H-B) connected to a belt-worn computer (Radxa, Rock Pi S), was used to collect BS at sampling frequency fs=16 kHz. The integrated microphone array was positioned according to the 9-quadrants abdominal maps (see Fig. A1 in Appendix A for further information). Different sizes were provided in the study to fit all participants, and stretchable fabric based on elastane was used to ensure optimal comfort and sensor skin interface. During the recording, participants were asked to relax on a lounger, while reading, using audio or video entertainment on a tablet, or sleeping. To avoid peristalsis overstimulation, participants could not drink caffeine-based beverages, e.g., coffee or tea, and laid down for most of the recording. Nevertheless, participants sat at a table to eat breakfast, and could often have conversation with study personnel. In addition, drinking water was allowed throughout the whole session.

BS annotation

Pairs of raters (experts in the field) annotated BS by visual and audio inspection of the signal using Audacity. Empirically, microphones placed on the stomach and small intestine collected showed most of BS events and largest Signal-to-Noise Ratio (SNR), therefore they were included in the annotation. Since IBD, especially UC, are often located between the small and large intestine, an additional microphone placed at the distal part of the large intestine was annotated for IBD patients and a subset of the healthy group. Every channel was labeled separately, since BS could occur at one or more channels depending on sound propagation in the abdomen [28]. Based on literature reported temporal features [33, 34] as well as preliminary annotation reviews, raters discussed and agreed on BS labeling approach: BS duration must be 18 ms, consecutive BS with SS interval < 100 ms were labeled as one event. BS with noisy temporal patterns were labeled as tentative and were excluded from the analysis. Depending on BS temporal occurrence, 1 h of audio could require 8-12 h per rater to label all BS events. Therefore, audio was annotated by one of the raters and labels were reviewed by another one. A subset comprising the first 30 min of audio from eight healthy participants and nine patients was used to evaluate annotation quality. Interrater agreement, i.e., Cohen's κ score, was used to analyze agreement on BS labels. Cohen's κ was computed on the subset with a time resolution of fs ≈ 0.06 ms.

Efficient-U-Net training and inference

Expert annotations were used to train an Efficient-U-Net (EffUNet) model for BS event spotting. The neural network architecture was adapted from U-Net model [35], original designed for biomedical image segmentation. UNet models are composed by an encoder and a decoder. The encoder extracts relevant features from the input data, and the decoder helps locating them on the original data by applying consecutive upsampling and concatenation operations (see Fig. 5). We selected EfficientNet-B2 [36] as encoder for our UNet model (hence the name EffUNet) since it showed promising results in spotting BS in continuous recordings [37]. EffUNet took as an input an audio clip of 10 s converted to log Mel-spectrogram representation and returned a binary time mask marking each spectrogram frame as either containing BS or not. EffUNet detection temporal resolution depends thus on spectrogram resolution. Figure 5 illustrates EffUNet architecture.

Recordings were preprocessed by applying a high-pass biquadratic filter (cutoff: 60 Hz) to remove offsets and split into non-overlapping audio segments of 10 s. Audio segments were subsequently converted to log Mel-spectrograms using a 25-ms sliding window with 10 ms stride (preprocessing: Hanning windowing) and 128 Mel-bins. Consequently, EffUNet spotting temporal resolution is 25 ms. The obtained audio spectrograms were 0-padded along the time dimension to 1056 frames.

Transfer learning was applied to EffUNet encoder, i.e., the encoder weights were initialized with EfficientNet-B2 pretrained on AudioSet dataset [38, 39], an audio dataset comprising more than 500 different sounds (including BS) and over 5000 h of audio split in 10-second audio clips sampled at 16 kHz. Since AudioSet only provides weak audio labels, no pretraining could be applied to EffUNet decoder, therefore He initialization [40] was used. Our previous experiments showed that transfer learning can improve BS spotting performance [37].

Leave-One-Participant-Out (LOPO) CV was used to train EffUNet on the expert annotated dataset. Following PSLA pipeline [38], EffUNet was trained for 25 epochs with an imbalanced batch size of 32. The initial learning rate of $1 \times 10^{-4}$ was subsequently reduced from the 6th epoch with a decay of 0.85 at each epoch. We used Adam optimizer [41] with $\beta 1 = 0.95$, $\beta 2 = 0.999$, and weight decay of $5 \times 10^{-7}$. The sum of cross-entropy loss and dice loss was used as loss function during the optimization. The model obtained at the end of the training was used to detect BS on the held-out participant recording.

Data augmentation was employed during the training to improve model generalization. Time-frequency masking [42] was applied to maximum 24 frequency bins and maximum 10% of spectrogram frames. White noise was randomly added to the input, and spectrogram frames could be shifted by up to 10 time bins.

To spot BS on the remaining unlabeled audio data, EffUNet was retrained on the expert annotated dataset using the above pipeline. BS were retrieved using EffUNet on one sensor on the stomach and two sensor on the small and large intestine respectively.

BS annotations were obtained from EffUNet binary masks as follows: A spotted BS event was defined as the set of consecutive overlapping spectrogram frames that were detected by EffUNet as containing BS.

Audio preprocessing and feature extraction

Audio was preprocessed using a 0-phase Butterworth band-pass filter of 8th order (passband frequency: 60-5000 Hz) following previous works on BS analysis [33, 34]. Using expert and EffUNet annotations, BS events were extracted from the recording and their duration was padded with the surrounding audio signal to fit the window length used for feature calculation. A sliding window of 32 ms length and a stride of 8 ms was used to calculate temporal, spectral, and perceptual features. The features obtained from each event across the sliding windows were subsequently averaged.

The recording was split into non-overlapping classification windows $S_i$ of duration $\delta$. BS occurring within the same classification window $S_i$ were grouped and mean and variance statistics was calculated for every BS feature. Therefore, classification windows not including BS events were excluded from the analysis. Additional features were included in the analysis, such as BS location, i.e. recording channel; number of BS per classification window (BS per unit time); and SS time interval. SS time interval was defined as the interval between consecutive BS within a classification window, and its mean and standard deviation (std) were considered in the feature analysis. For the classification task, only the best features were used for the training. We therefore selected the best BS features based on feature mutual information [43].

The obtained classification windows $S_i$ were subsequently labeled as "healthy" if they were collected from the healthy population, otherwise they were marked as "IBD". For the IBD type classification task, classification windows $S_i$ from the patient group were relabeled as "UC" if they were recorded from UC patients and "CD" if they were obtained from CD patients.

Evaluation metrics

IBD detection performance was evaluated across all k CV folds by computing the AUROC, sensitivity and specificity. From the results of each testing fold, performance statistics were calculated.

The IBD detection model score was subsequently compared with the stool and blood inflammation biomarkers to analyze the correlation between the IBD class probability from the applied GBC model and disease activity across all patients. Spearman's r correlation coefficient was used to investigate the relationship between our model IBD score and biomarkers levels. The classifier performance in detecting IBD patients in the study group was evaluated by calculating accuracy, sensitivity and specificity scores on the merged predictions obtained for every participant with majority voting. Mean and std of AUROC were analyzed to find the optimal classification window duration $\delta$, i.e., minimum amount of audio to analyze, to detect IBD, yielding the largest mean AUROC and smallest std AUROC across all experiments.

**Example 1**

**Results**

BS annotation and data preprocessing

Pairs of raters annotated recordings from 27 participants (18 healthy, 9 IBD patients, 3 patients in remission). The agreement was evaluated on the first 30 min of audio from eight healthy participants and nine patients. The evaluation yielded a substantial agreement, with Cohen's $\kappa$ score of 0.70 on the healthy group and 0.73 on the patient group. On approx. 136 h of audio, 11482 BS were annotated by the raters. The full dataset comprising 45 participants was subsequently analyzed with the inventors spotting model based on Efficient-U-Net (EffUNet) deep neural network to detect BS. On approx. 260 h of audio, EffUNet identified 22442 BS. Figure 1 shows the amount of per participant annotated BS, by expert raters and EffUNet. On the annotated dataset, our spotting model and expert raters identified a similar amount of BS. The recording from each channel was split into non-overlapping classification windows Si of duration $\delta=10$ min. In total, we obtained in total 704 classification windows (234 $S_i$ from the patient group) on the expert annotated dataset and 1236 classification windows (682 $S_i$ from the patient group) on the full dataset analyzed with EffUNet.

Feature selection and IBD classification window detection

For each classification window, mean and variance statistics was calculated from the acoustic features of the BS included in Si. In addition, BS location, i.e., recording channel, BS events per classification window (BS per unit time), and SS time interval were extracted for each classification window $S_i$. Based on the mutual information score, the best obtained features were selected to train a GBC. Among the BS features, the mean of Mel-frequency cepstral coefficients (MFCCs) yielded the highest mutual information score, along with BS location, the mean and variance of spectral flatness, and the mean of spectral complexity, zero crossing rate, signal energy, strong peak ratio (i.e., ratio between the maximum peak in a spectrum and the peak bandwidth), and signal inharmonicity. To minimize the feature set used for the IBD prediction, the inventors selected the mean of 11 MFCCs (3rd to 13th coefficients) to train our classifier. The first two MFCCs were not included in the feature set as the audio recording was pre-processed with a high-pass filter, thus removing the spectral content included in those coefficients..

The IBD classification performance on the annotated dataset of 27 participants is shown in Fig. 2a-b. Stratified group 9 fold CV was used to evaluate the classifier, i.e., each validation fold contained classification windows $S_i$ from one patient only. GBC achieved mean AUROC (area under ROC) of 0.88 (std=0.08), mean sensitivity of 0.81 (std=0.12) and mean specificity of 0.88 (std=0.09) across all CV experiments when analyzing BS events marked by experts. The same performance was achieved on the 27-participant dataset when analyzing BS events detected by EffUNet, with mean AUROC of 0.90 (std=0.04), mean sensitivity of 0.88 (std=0.13), and mean specificity of 0.84 (std=0.08). Figure 2c shows the classification performance on the full dataset annotated by EffUNet. The model was trained and tested using stratified group 21 fold, i.e., each validation fold included data from approx. one patient and one healthy participant. A mean AUROC of 0.83 (std=0.11) was achieved across all CV experiments, with mean sensitivity of 0.80 (std=0.15) and mean specificity of 0.83 (std=0.13). Compared to the annotated dataset of 27 participants, the AUROC std is higher on the full dataset including more IBD patients.

Correlation with IBD activity

For each patient, the inventors obtained a classification score by averaging the IBD class probability across all patient classification windows $S_i$. We subsequently analyzed the correlation between the classification score and inflammation biomarker levels with Spearman's r. The relationship obtained between classification scores and biomarker level distributions are shown in the Quantile-Quantile (QQ) plots in Fig. 3. QQ plots were estimated for detected and missed IBD patients. On the expert annotated dataset of 27 participants, very weak correlation was found with CRP (r=-0.12) and fCP (r=-0.02), while correlation with leukocyte counts was moderate (r=0.47). When using EffUNet labels on the 27-participant dataset, the analysis identified a very weak correlation with CRP (r=-0.03) and leukocyte counts (r=-0.10), and a weak correlation with fCP (r=0.22). Correlation between IBD class probability and CRP on the full dataset was very weak (r=-0.17). Correlation with fCP and leukocyte counts was very weak (r=0.0.02 for fCP, r=0.-0.01 for leukocyte counts) too.

IBD prediction with majority voting

The classification window predictions were merged for every study participant by employing majority voting, obtaining thus a single prediction per participant. The classification yielded an accuracy of 0.93 on the expert annotated dataset of 27 participants, with a sensitivity of 0.89 and a specificity of 0.94. On the annotated dataset when using EffUNet labels, the classification yielded an accuracy, sensitivity, and specificity of 1. On the full dataset annotated by EffU-Net, GBC achieved an accuracy of 0.93, with sensitivity=0.88 and specificity=1.

Minimum recording duration required to detect IBD

Subsequently, the minimum amount of audio recording necessary to detect IBD was investigated. The duration $\delta$ of classification windows $S_i$ used to split and analyze the recording was swept from 1 s to 10 min. Both annotated and full dataset were employed in the analysis. For each classification window duration $\delta$, GBC was retrained and evaluated using BS features and CV approach described herein. The results are shown in Fig. 4. For all datasets, the mean AUROC increases when longer classification windows $S_i$ are used for the detection. In particular, the best mean AUROC is achieved for $\delta$=10 min. Along with $\delta$ increase, the AUROC std decreases and is minimum for $\delta$=10 min.

**Discussion**

The diagnosis and monitoring of IBD relies nowadays on a combination of clinical, imaging and biochemical assessments. Due to the variety of investigations needed and, consequently, the time required to perform the investigations, IBD diagnosis could be affected by delays [22]. As most diagnostics, e.g., inflammation biomarkers, are not IBD-specific, more invasive techniques are performed to confirm the diagnosis, e.g., endoscopy. Although endoscopy is considered the gold standard to ultimately diagnose IBD as well as assess disease state, the technique is costly, invasive, and can cause patient discomfort. The inventors herein aimed to provide physicians a new approach to non-invasively detect IBD from continuous abdominal recordings. The inventors investigated BS-derived features and trained a gradient boosting classifier (GBC) to predict 10 min audio recordings captured from IBD patients vs. healthy audio data. In the present study, a low-cost wearable device was used to collect BS across different digestive phases inconspicuously. Compared to existing diagnostics, the present approach is inexpensive and can monitor digestion throughout the whole process, avoiding repeated and uncomfortable abdominal assessments. The present examples show that the present method can successfully be employed as a bowel disease, e.g., IBD, screening test, and is able to be used as a telemedicine solution, minimizing thus the amount of patient hospital visits.

The present analysis on BS features shows that IBD can be acoustically detected with a mean sensitivity and a mean specificity of 0.88 across the recordings annotated by expert raters (see Fig. 2a). On the same subset of participants, the GBC achieved the similar performance when using EffUNet BS annotations, confirming the previous results.

To monitor IBD less invasively, various biomarkers from blood, stool or urine samples were proposed [5]. In this study, we biochemically assessed IBD activity using fCP, CRP and leukocyte counts. The inventors analyzed the correlation between the per-patient mean IBD class probability and the biomarkers levels to test our approach as a potential prognosis monitoring tool. Correlation was highest between IBD class probability and leukocyte counts. When comparing IBD probability with other biomarkers, the correlation was weak or very weak depending on the dataset analyzed.

The Inventor's analysis showed that the present method can detect individuals with active inflammation, resulting in increased leukocyte counts and fCP concentration. Because CRP concentration rapidly changes in case of acute inflammation and has shorter half time compared to other biomarkers [25], the inventors hypothesized that BS and CRP levels change at a different pace overtime. Moreover, CRP concentration changes highly depend on IBD disease, e.g. CRP response is more evident in CD than in UC cases [25]. In addition, IBD biochemical inflammation assessment can be challenging, as biomarkers concentration ranges to determine remission/relapse may vary depending on the used test kits, patient ethnicity/age, and physician experience [26]. For instance, fCP concentration between 50 and 150 $\mu$g/g could be considered insufficient to assess IBD remission and the test might need to be repeated. In the present study, 5 patients had fCP levels in 50-150 $\mu$g/g range. Patients having fCP concentration above 250 $\mu$g/g were considered having active inflammation.

BS pattern analysis could be used not only to detect IBD but also to monitor the disease during the treatment. Compared IBD biochemical assessment, the monitoring based on BS could be done continuously and remotely, allowing physicians monitor the disease response to treatment even outside the regular hospital visits.

The method has been further demonstrated as a valuable screening test by analyzing the classification performance on the study population besides the classification window dataset. To obtain one IBD prediction per participant, the inventors merged the classification window predictions for each participant with majority voting. Regardless of the BS annotation method, the IBD detection using the computation of MFCC-features from the recorded bowl sounds as described herein yielded an accuracy 0.91.

As for diagnostic tools, it is important to assess the minimum amount of data necessary to detect IBD. In standard manual auscultation, it is recommended listening to BS for a few minutes [14]. However, when no BS are heard, auscultation should be prolonged up to 10 min, to maximize BS event amount [15]. The inventor's performance analysis over classification window duration $\delta$ confirmed common clinical practice, as the highest mean AUROC and lowest std AUROC was obtained for $\delta$ = 10 min (see Fig. 4). Although classification window duration < 1 min yielded mean AUROC above 0.74 regardless of the used BS annotation tool, GBC performance is more variable, especially when EffUNet annotations are employed.

Although CD and UC manifest similar symptoms across patients, they differently affect the GI luminal walls. It is then necessary determining the IBD type to carefully plan the treatment. Clinical guidelines recommend to perform an endoscopic exam and analyze at least two biopsy samples from the inflamed regions to determine IBD type [4]. The primary goal of this work was to design an AI-based method to acoustically detect and distinguish patients with gastrointestinal diseases, such as IBD, from healthy controls. The inventors propose their approach as a (preliminary) test to determine whether potential IBD patients should undergo more invasive exams, i.e., endoscopy, to confirm diagnosis. They further investigated BS potential in diagnosing the two main IBD types and retrained GBC to predict CD vs UC patients based on most relevant BS features.

Moreover, BS recording as described herein could be combined with imaging techniques to establish source location ground truth, e.g. [30], and develop source localization methods leveraging on microphone arrays.

In conclusion, the present work presents an analysis of BS features collected across IBD patients with different disease condition as well as healthy control. The most relevant features were selected and an AI was trained to detect audio classification windows of 10 min, collected from IBD vs. healthy. The present method can detect IBD patients with mean AUROC above 0.83 regardless of the annotation tool employed to mark BS in the audio recordings.

The present analysis of correlation between IBD probability and inflammation biomarker levels showed that there was a weak to moderate correlation depending on the biomarker and BS annotation tool. The present method returned IBD probability > 0.50 for all patients, regardless of the disease condition. In particular, IBD was predicted by GBC with accuracy 0.91. The results show that this method could be potentially employed in clinical practice as a gastrointestinal disease and particularly an IBD screening test prior to use more invasive investigations such as endoscopy.

**Example 2**

Example 1 demonstrates that IBD classification using the proposed method may efficiently support IBD diagnosis in a screening test, when Mel Frequency Cepstral Coefficient (MFCC)-features are used by an artificial intelligence (AI) to determine the likelihood a subject suffering from a bowel disease or not.

In order to evaluate further the contribution of Mel Frequency Cepstral Coefficient (MFCC)-features versus alternative features (such as mean energy, BS location etc.) feature selection experiments were performed using the data of Example 1 and comparing the results using different feature sets:

Feature selection experiments

The following table shows a comparison of different feature sets tested with an embodiment of the present method.

Table 1: Full dataset (BS events retrieved with Efficient-U-Net)

| Number of features | Features | Mean ROC AUC (cross-validation experiments) | St.dev. ROC AUC (cross-validation experiments) |
|---|---|---|---|
| 7 | mean energy, mean **MFCCs** (1st, 3rd, 9th, 13th components), variance 13th **MFCC,** BS location | 0.83 | 0.18 |
| 7 | mean max magnitude frequency, mean **MFCCs** (1st, 2nd, 4th, 7th, 9th components), variance **MFCCs** (13th component) | 0.85 | 0.16 |
| 13 | 13 **MFCCs,** mean | 0.87 | 0.14 |
| 20 | 10 **MFCCs,** mean and variance | 0.85 | 0.17 |
| 24 | 12 **MFCCs,** mean and variance | 0.86 | 0.14 |
| 40 | 20 **MFCCs,** mean and variance | 0.86 | 0.14 |
| 60 | 30 **MFCCs,** mean and variance | 0.86 | 0.14 |
| 80 | 40 **MFCCs,** mean and variance | 0.88 | 0.14 |
| 17 | Alternative features: Skewness of spectral centroid, spectral flatness, strong peak ratio, spectral spread, max magnitude frequency, spectral complexity, zero crossing rate, 10 spectral subband energy | 0.68 | 0.12 |
| 7 | Alternative features: Skewness of spectral centroid, spectral flatness, strong peak ratio, spectral spread, max magnitude frequency, spectral complexity, zero crossinq rate | 0.64 | 0.16 |

(continued)

| Number of features | Features | Mean ROC AUC (cross-validation experiments) | St.dev. ROC AUC (cross-validation experiments) |
|---|---|---|---|
| 34 | Alternative features: Skewness and kurtosis of spectral centroid (extracted as alternative statistics (i.e., statistical measure) of the BS feature distributions), spectral flatness, strong peak ratio, spectral spread, max magnitude frequency, spectral complexity, zero crossing rate, 10 subband energy | 0.66 | 0.10 |

Table 2: Annotated dataset (BS retrieved manually)

| Number of features | Features | Mean ROC AUC (cross-validation experiments) | St.dev. ROC AUC (cross-validation experiments) |
|---|---|---|---|
| 9 | 9 **MFCCs,** mean | 0.94 | 0.05 |
| 10 | 10 **MFCCs,** mean | 0.94 | 0.05 |
| 12 | 12 **MFCCs,** mean | 0.94 | 0.05 |
| 13 | 13 **MFCCs,** mean | 0.94 | 0.05 |
| 20 | 20 **MFCCs,** mean | 0.95 | 0.04 |
| 60 | 30 **MFCCs,** mean and variance | 0.95 | 0.03 |
| 80 | 40 **MFCCs,** mean and variance | 0.95 | 0.04 |

In the experiments shown in Table 2, higher ROC-AUC values were achieved, as the dataset used for said experiments comprised only BS events annotated manually by experts, which reduced the likelihood of false positives (i.e., events that were wrongly marked as "BS events"). In the full dataset used for the experiments shown in Table 1, also an EffUNet was used for BS event detection and the audio data comprised data of participants that were not manually annotated and therefore not used for the DNN training.

The present data demonstrate that in some embodiments the advantageous feature set for the screening test may comprise 5, 7, 10 MFCCs or more (mean values).

According to the present experiments, some of the MFCCs components could be omitted and replaced with alternative spectral/perceptual features without affecting the model performance. However, including at least one MFCCs, preferably 5 MFCCs, 7 MFCCs, 10 MFCCs or more consistently demonstrated improved results over an analysis using AI, which did not include computing at least one MFCC. For instance, as demonstrated in Table 1 using 17 features including Skewness of spectral centroid, spectral flatness, strong peak ratio, spectral spread, max magnitude frequency, spectral complexity, zero crossing rate, 10 spectral subband energy, but no MFCCs only yielded a Mean ROC AUC (cross-validation experiments) of 0.68. On the contrary using at least 5 MFCCs (possibly accompanied by further features such as mean energy or a BS location) consistently yielded a Mean ROC AUC (cross-validation experiments) of 0,83. Using more than 20 MFCCs even increased the Mean ROC AUC to 0.85 or more. The present examples thus demonstrate, how MFCCs features may be advantageously used the context of the present invention to predict bowel diseases, wherein preferably a one-channel analysis is performed, and/or an analysis over a statistical window (e.g., 10 min of audio recording).

A non-limiting list of features of which one or more were used in the context of the present method in addition to one or more MFCC(-feature) is as follows:

Spectral Features

Mel Frequency Cepstral Coefficients (MFCCs) (Signal frequency bands representation according to the mel-scale, i.e. human auditory system. In this example, at least the first 13 coefficients were employed for the classification).

Spectral centroid (The center of mass of a signal spectrum. Perceptually, the spectral centroid is related to sound brightness).

Spectral complexity (A measurement of how much information is needed to estimate the signal spectrum, it is based on the amount of spectrum peaks).

Spectral flatness (How much a signal spectrum is noise-like, e.g. white noise has value 1, or similar to a pure tone sound, which has value 0).

Spectral spread (It defines the signal spectrum variance around the spectral centroid).

Strong peak ratio (The ratio between the maximum peak's magnitude and the peak band- width in a spectrum. The higher the ratio, the more pronounced is the peak. We estimated the ratio according to Gouyon and Herrera [44].)

Maximum magnitude frequency (measures the frequency having the largest magnitude in a spectrum).

Temporal Features

Signal energy

Zero crossing rate (Number of time the signal change from positive to negative and vice- versa per unit time).

Perceptual Features

Dissonance (Perceptual roughness of an audio signal based on the spectrum peaks. The dissonance may be estimated, e.g., according to Plomp and Levelt [45]).

Inharmonicity (How much harmonic a signal is based on the signal fundamental frequency and other spectrum peaks).

Signal pitch (Signal fundamental frequency. The Yin algorithm [46] was used for the estimation).

Other Features

BS location (It is defined as the location of the sensor that recorded the BS).

BS per unit time (Number of BS events occurring within a classification window, i.e. portion of audio recording of duration $\delta$).

Sound-to-Sound (SS) interval (Time interval in seconds occurring between consecutive BS events within a classification window).

**Example 3**

An exemplary experimental workflow of one embodiment of the present method comprised the following steps:

2. Continuous audio recording (auscultation) of the digestion, i.e. Bowel Sound (BS) data collection.
3. Audio preprocessing.

   i. High-pass biquadratic filter (frequency cutoff 60 Hz).
   ii. BS event spotting: either manually (expert annotation) or with AI model (Efficient-U-Net).

4. Feature extraction for each spotted BS.

   i. Band-pass 8th order Butterworth filter (passband 60-5000 Hz).
   ii. If BS duration is < 32 ms or not a multiple of 32 ms, BS signal is padded before onset and after offset with audio data recorded before and after the spotted BS events until new BS duration is equal to 32 ms or a multiple of 32 ms.
   iii. Power spectrogram calculation of BS event using a sliding window of 32 ms with 8 ms stride (This is the sliding window procedure used to extract BS features. When calculating and testing other BS features, we always split BS events into windows of 32 ms duration with 8 ms stride). Hann windowing is used for the spectrogram calculation, i.e. every sliding window is multiplied by Hann window before calculating spectrogram.
   iv. Apply Mel-filterbank to the spectrogram to obtain Mel-spectrogram.

      a) Convert frequency from Hz to Mel scale according to following equation:

$$m = 2595 \log_{10}\left(1 + \frac{f}{700}\right)$$

,

Where $f$ is the frequency in Hz and $m$ is the frequency in mels.
      b) Divide frequency into discrete, overlapping bins according to Mel-filterbanks frequency bands.

c) Multiply each Mel-filterbank (triangular window) by the corresponding power spectrogram frequency bin and sum the power spectrogram of all frequencies included in the bin.

v. Convert the Mel-spectrogram amplitude to decibel (dB) scale.

vi. Apply type 2 discrete cosine transform to the Mel-spectrogram to obtain Mel-Frequency Cepstrum Coefficients (MFCCs). Select first 20 MFCCs.

vii. Average MFCCs across all 32-ms sliding windows used to calculate BS Mel-spectrogram, obtaining thus 20 MFCCs per BS event.

5. Divide continuous recording into classification windows of 10 minutes. For each 10-minute classification window, calculate feature statistics (mean and variance of MFCCs) of BS events occurring within the 10-minute window.

6. Classify (with Gradient Boosting Classifier) each 10-minute classification window as either being recorded from an IBD patient or a healthy participant.

Dataset

For each study participant, 1 h of audio before breakfast (fasting phase) and 1 h of audio after breakfast (postprandial phase) were recorded.

In total 45 participants were recruited: 21 healthy participants and 24 IBD patients. Out of 21 healthy participants, 18 individuals were manually analyzed to annotate BS events. Out of 24 patients, 9 individuals were manually analyzed to annotate BS events.

Automatic spotting method (Efficient-U-Net)

The annotated dataset of 18 healthy participants and 9 IBD patients was used to train the Efficient-U-Net BS spotting model, using Leave-One-Participant-Out (LOPO) cross-validation. With LOPO cross-validation, Efficient-U-Net was trained on 26 annotated participants and tested on the held-out participant. Efficient-U-Net spotting results (i.e. start to end of each retrieved BS) were subsequently used to extract features from the spotted events for the screening test (IBD classifier). Efficient-U-Net was subsequently trained on all manually annotated data (27 participants) to spot BS events on the remaining, non-annotated dataset.

Screening test evaluation

Use Group K Fold cross-validation to train and test an AI model (Gradient Boosting Classifier) on the obtained classification windows. Use Group K Fold cross-validation to split the dataset in trainset (=training set) and test set based on the participant: For each fold, all classification windows of one participant can either be in the trainset (=training set) or test set, not in both sets at the same time (i.e. no data leakage).

The Efficient-U-Net (spotting model) and Gradient Boosting Classifier (screening test model) are two steps of the workflow independent from each other, i.e. one can replace Efficient-U-Net spotting results (i.e. start to end of each retrieved BS) with the expert manual BS annotations (where available).

**Example 4**

The inventors further analyzed the GBC performance in distinguishing IBD and healthy controls. They randomly resampled from the recordings of each study participant audio segments $N_i$ containing only environmental noise from the reference channel placed on the esophagus. The selected noise segments had variable duration, following the same duration distribution of the expected annotated BS events. Both noise events $N_i$ and BS events $e_j$ were preprocessed by applying a bandpass $8^{th}$ Butterworth filter with bandwidth 60-5000 Hz.

From each noise segment $N_i$ the same features were extracted as used to train the GBC classifier, i.e., 11 MFCCs (e.g., MFCCs coefficients from 3 to 1). As for BS events, noise events falling in the same 10-minute classification window were grouped together and feature statistics (mean, variance) was estimated from the event feature distribution.

The GBC was trained on classification windows $S_i$ containing only BS using the same Group K Fold Cross-Validation (CV) approach and tested on the set containing classification windows having only noise segments $N_i$. As for the Examples 1 and 2, SMOTE resampling was applied on the trainset of each fold to balance the classification windows extracted from healthy controls and IBD patients. The GBC performance was evaluated across all k CV folds by computing the AUROC, sensitivity and specificity. From the results of each testing fold, performance statistics were calculated.

Table 3: GBC performance on noise classification windows $N_i$ randomly sampled from the reference microphone placed on the esophagus.

| | Mean ROC AUC (cross-validation experiments) | St.dev. ROC AUC (cross-validation experiments) |
|---|---|---|
| Annotated dataset, expert BS labels | 0.60 | 0.17 |
| Annotated dataset, EffUNet BS annotations | 0.60 | 0.10 |
| Full dataset, EffUNet BS annotations | 0.60 | 0.25 |

The low performance of the GBC classifier on the validation set comprising features derived from non-BS events confirms the reliability of the method according to the invention to distinguish IBD patients and healthy controls using BS events and related features.

REFERENCES

[1] Seyedian, S.S., Nokhostin, F., Malamir, M.D.: A review of the diagnosis, prevention, and treatment methods of inflammatory bowel disease. Journal of medicine and life 12(2), 113 (2019).

[2] Baldassano, R.N., Piccoli, D.A., et al.: Inflammatory bowel disease in pediatric and adolescent patients. Gastroenterology Clinics of North America 28(2), 445-458 (1999).

[3] Hammer, T., Langholz, E.: The epidemiology of inflammatory bowel disease: Balance between East and West? A narrative review. Digestive Medicine Research 3(0) (2020) https://doi.org/10.21037/dmr-20-149 .

[4] Maaser, C., Sturm, A., Vavricka, S.R., Kucharzik, T., Fiorino, G., Annese, V., Calabrese, E., Baumgart, D.C., Bettenworth, D., Borralho Nunes, P., Burisch, J., Castiglione, F., Eliakim, R., Ellul, P., Gonz'alez-Lama, Y., Gordon, H., Halligan, S., Katsanos, K., Kopylov, U., Kotze, P.G., Krustinṣ, E., Laghi, A., Limdi, J.K., Rieder, F., Rimola, J., Taylor, S.A., Tolan, D., Rheenen, P., Verstockt, B., Stoker, J.: ECCO-ESGAR Guideline for Diagnostic Assessment in IBD Part 1: Initial diagnosis, monitoring of known IBD, detection of complications. Journal of Crohn's and Colitis 13(2), 144-164 (2018) https://doi.org/10.1093/ecco-jcc/jjy113 https://academic.oup.com/ecco-jcc/articlepdf/13/2/144/27790815/jjy 113.pdf.

[5] Wagatsuma, K., Yokoyama, Y., Nakase, H.: Role of biomarkers in the diagnosis and treatment of inflammatory bowel disease. Life 11(12), 1375 (2021).

[6] Shergill, A.K., Lightdale, J.R., Bruining, D.H., Acosta, R.D., Chandrasekhara, V., Chathadi, K.V., Decker, G.A., Early, D.S., Evans, J.A., Fanelli, R.D., et al.: The role of endoscopy in inflammatory bowel disease. Gastrointestinal endoscopy 81(5), 1101-1121 (2015) [7]

Kohli, A., Holzwanger, E.A., Levy, A.N.: Emerging use of artificial intelligence in inflammatory bowel disease. World Journal of Gastroenterology 26(44), 6923 (2020).

[8] Kilcoyne, A., Kaplan, J.L., Gee, M.S.: Inflammatory bowel disease imaging: Current practice and future directions. World Journal of Gastroenterology 22(3), 917-932 (2016) https://doi.org/10.3748/wjg.v22.i3.917.

[9] Stafford, I.S., Gosink, M.M., Mossotto, E., Ennis, S., Hauben, M.: A Systematic Review of Artificial Intelligence and Machine Learning Applications to Inflammatory Bowel Disease, with Practical Guidelines for Interpretation. Inflammatory Bowel Diseases 28(10), 1573-1583 (2022) https://doi.org/10.1093/ ibd/izac115.

[10] Han, L., Maciejewski, M., Brockel, C., Gordon, W., Snapper, S.B., Korzenik, J.R., Afzelius, L., Altman, R.B.: A probabilistic pathway score (props) for classification with applications to inflammatory bowel disease. Bioinformatics 34(6), 985-993 (2018).

[11] Chierici, M., Puica, N., Pozzi, M., Capistrano, A., Donzella, M.D., Colangelo, A., Osmani, V., Jurman, G.: Automatically detecting crohn's disease and ulcerative colitis from endoscopic imaging. BMC Medical Informatics and Decision Making 22(6), 1-11 (2022).

[12] Stidham, R.W., Liu, W., Bishu, S., Rice, M.D., Higgins, P.D.R., Zhu, J., Nallamothu, B.K., Waljee, A.K.: Performance of a Deep Learning Model vs Human Reviewers in Grading Endoscopic Disease Severity of Patients With Ulcerative Colitis. JAMA Network Open 2(5), 193963-193963 (2019) https://doi.org/10.1001/jamanetworkopen. 2019.3963.

[13] Fritz, D., Weilitz, P.B.: Abdominal Assessment. Home Healthcare Now 34(3), 151- 155 (2016) https://doi.org/10.1097/NHH.

[14] Reuben, A.: Examination of the abdomen. Clinical Liver Disease 7(6), 143-150 (2016) https://doi.org/10.1002/cld.556.

[15] Baid, H.: A critical review of auscultating bowel sounds. British journal of nursing 18(18), 1125-1129 (2009) [16] Huang, Y., Song, I., Rana, P., Koh, G.: Fast diagnosis of bowel activities, pp. 3042-3049. IEEE, ??? (2017). https://doi.org/10.1109/ IJCNN.2017.7966234.

[17] Craine, B.L., Silpa, M., O'Toole, C.J.: Computerized auscultation applied to irritable bowel syndrome. Digestive diseases and sciences 44, 1887-1892 (1999).

[18] Craine, B.L., Silpa, M.L., O'Toole, C.J.: Enterotachogram analysis to distinguish irritable bowel syndrome from crohn's disease. Digestive diseases and sciences 46, 1974-1979 (2001).

[19] Ranta, R., Louis-Dorr, V., Heinrich, C., Wolf, D., Guillemin, F.: Principal component analysis and interpretation of bowel sounds, vol. 3, pp. 227-230. IEEE, ??? (2004). https:// doi.org/10.1109/IEMBS.2004.1403133.

[20] Du, X., Allwood, G., Webberley, K.M., Inderjeeth, A.-J., Osseiran, A., Marshall, B.J.: Noninvasive diagnosis of irritable bowel syndrome via bowel sound features: Proof of concept. Clinical and translational gastroenterology 10(3) (2019).

[21] Spiegel, B.M., Kaneshiro, M., Russell, M.M., Lin, A., Patel, A., Tashjian, V.C., Zegarski, V., Singh, D., Cohen, S.E., Reid, M.W., et al.: Validation of an acoustic gastrointestinal surveillance biosensor for postoperative ileus. Journal of Gastrointestinal Surgery 18, 1795- 1803 (2014).

[22] Cross, E., Saunders, B., Farmer, A.D., Prior, J.A.: Diagnostic delay in adult inflammatory bowel disease: A systematic review. Indian Journal of Gastroenterology 42(1), 40-52 (2023) https://doi.org/10.1007/ s12664-022-01303-x.

[23] Kam, J., Taylor, D.M.: Obesity significantly increases the difficulty of patient management in the emergency department. Emergency Medicine Australasia 22(4), 316-323 (2010) https://doi.org/10.1111/j.1742-6723. 2010.01307.x

[24] Zhao, Z., Li, F., Xie, Y., Wu, Y., Wang, Y.: BS-Monitor: Noise-Resistant Bowel Sound Monitoring Via Earphones. IEEE Transactions on Mobile Computing, 1-15 (2023) https://doi.org/10.1109/TMC.2023.3270926.

[25] Vermeire, S., Van Assche, G., Rutgeerts, P.: Laboratory markers in IBD: Useful, magic, or unnecessary toys? Gut 55(3), 426-431 (2006) https://doi.org/10.1136/gut.2005.069476 [26] Bjarnason, I.: The Use of Fecal Calprotectin in Inflammatory Bowel Disease. Gastroenterology & Hepatology 13(1), 53-56 (2017).

[27] Guindi, M., Riddell, R.H.: Indeterminate colitis. Journal of Clinical Pathology 57(12), 1233-1244 (2004) https://doi.org/10.1136/ jcp.2003.015214.

[28] Ranta, R., Louis-Dorr, V., Heinrich, C., Wolf, D., Guillemin, F.: Towards an acoustic map of abdominal activity. In: Proceedings of the 25th Annual International Conference of the IEEE Engineering in Medicine and Biology Society (IEEE Cat. No.03CH37439), vol. 3, pp. 2769-27723 (2003). https://doi.org/10. 1109/IEMBS.2003.1280491.

[29] Dimoulas, C.A.: Audiovisual Spatial-Audio Analysis by Means of Sound Localization and Imaging: A Multimedia Healthcare Framework in Abdominal Sound Mapping. IEEE Transactions on Multimedia 18(10), 1969- 1976 (2016) https://doi.org/10.1109/TMM. 2016.2594148.

[30] Saito, S.-N., Otsuka, S., Zenbutsu, S., Hori, S., Honda, M., Nakagawa, S.: Generation mechanisms of bowel sounds by simultaneous measurements of X-ray fluoroscopy and bowel sounds, pp. 1593-1596. IEEE, ??? (2021). https://doi.org/10.1109/ EMBC46164.2021.9629486

[31] Friedman, J.H.: Greedy function approximation: a gradient boosting machine. Annals of statistics, 1189-1232 (2001).

[32] Baronetto, A., Graf, L.S., Fischer, S., Neurath, M.F., Amft, O.: Gastro Digital Shirt: A Smart Shirt for Digestion Acoustics Monitoring. In: ISWC '20: Proceedings of the 2020 International Symposium on Wearable Computers, pp. 17-21. ACM, Virtual Conference (2020). https://doi.org/10.1145/ 3410531.3414297 . https://dl.acm.org/doi/ 10.1145/3410531.3414297 Accessed 2020-0910.

[33] Dimoulas, C.A., Papanikolaou, G.V., Petridis, V.: Pattern classification and audiovisual content management techniques using hybrid expert systems: A video-assisted bioacoustics application in Abdominal Sounds pattern analysis. Expert Systems with Applications 38(10), 13082-13093 (2011) https://doi.org/10.1016/j.eswa.2011.04.115 .

[34] Du, X., Allwood, G., Webberley, K.M., Osseiran, A., Wan, W., Volikova, A., Marshall, B.J.: A mathematical model of bowel sound generation. The Journal of the Acoustical Society of America 144(6), 485-491 (2018) https://doi.org/ 10.1121/1.5080528.

[35] Ronneberger, O., Fischer, P., Brox, T.: UNet: Convolutional Networks for Biomedical Image Segmentation. In: Navab, N., Hornegger, J., Wells, W.M., Frangi, A.F. (eds.) Medical Image Computing and ComputerAssisted Intervention - MICCAI 2015 vol. 9351, pp. 234-241. Springer International Publishing, Cham (2015).

[36] Tan, M., Le, Q.V.: EfficientNet: Rethinking Model Scaling for Convolutional Neural Networks (2019). https://doi.org/10.48550/ arXiv.1905.11946 . https://arxiv.org/abs/ 1905.11946v5 Accessed 2022-08-03.

[37] A. Baronetto, L. S. Graf, S. Fischer, M. F. Neurath, and O. Amft, "Segment-based Spotting of Bowel Sounds using Pretrained Models in Continuous Data Streams," IEEE Journal of Biomedical and Health Informatics, vol. 27, no. 7, pp. 3164-3174, Jul. 2023, doi: 10.1109/JBHI.2023.3269910.

[38] Gong, Y., Chung, Y.-A., Glass, J.: PSLA: Improving Audio Tagging with Pretraining, Sampling, Labeling, and Aggregation (2021). https://doi.org/10.1109/TASLP. 2021.3120633 . https://arxiv.org/abs/2102. 01243v3 Accessed 2022-08-03

[39] Gemmeke, J.F., Ellis, D.P.W., Freedman, D., Jansen, A., Lawrence, W., Moore, R.C., Plakal, M., Ritter, M.: Audio Set: An ontology and human-labeled dataset for audio events. In: 2017 IEEE International Conference on Acoustics, Speech and Signal Processing (ICASSP), pp. 776-780. IEEE, ??? (2017). https://doi.org/10.1109/ICASSP. 2017.7952261 . http://ieeexplore.ieee.org/ document/7952261/ Accessed 2022-08-03.

[40] He, K., Zhang, X., Ren, S., Sun, J.: Delving Deep into Rectifiers: Surpassing Human Level Performance on ImageNet Classification (2015). https://doi.org/10.48550/ arXiv.1502.01852 . https://arxiv.org/abs/ 1502.01852v1 Accessed 2022-08-03.

[41] Kingma, D.P., Ba, J.: Adam: A Method for Stochastic Optimization (2014). https: //doi.org/10.48550/ar-Xiv.1412.6980 . https: //arxiv.org/abs/1412.6980v9.

[42] Park, D.S., Chan, W., Zhang, Y., Chiu, C.-C., Zoph, B., Cubuk, E.D., Le, Q.V.: SpecAugment: A Simple Data Augmentation Method for Automatic Speech Recognition (2019). https: //doi.org/10.21437/Interspeech.2019-2680 .https://arxiv.org/abs/1904.08779v3.

[43] Ross, B.C.: Mutual information between discrete and continuous data sets. PloS one 9(2), 87357 (2014).

[44] Gouyon, F., Herrera, P.: Exploration of techniques for automatic labeling of audio drum tracks instruments. In: Proceedings of MOSART: Workshop on Current Directions in Computer Music (2001).

[45] Plomp, R., Levelt, W.J.M.: Tonal consonance and critical bandwidth. The journal of the Acoustical Society of America 38(4), 548-560 (1965).

[46] De Cheveigné, A., Kawahara, H.: Yin, a fundamental frequency estimator for speech and music. The Journal of the Acoustical Society of America 111(4), 1917-1930 (2002)

[47] Digestive-system 3D Model by Genesis1. https://sketchfab.com/models/ 2db2c9090e1 d4e1891826958398d324f/.

[48] J. Ficek, K. Radzikowski, J. K. Nowak, O. Yoshie, J. Walkowiak, and R. Nowak, "Analysis of Gastrointestinal Acoustic Activity Using Deep Neural Networks," Sensors, vol. 21, no. 22, Art. no. 22, Nov. 2021, doi: 10.3390/s21227602.

[49] Mahalakshmi, P. "A Review On Voice Activity Detection And Mel-Frequency Cepstral Coefficients For Speaker Recognition (Trend Analysis)." Asian Journal of Pharmaceutical and Clinical Research 9 (2016): 360.

[50] A. Abdulsatar, V. Davydov, V. Yushkova, A. P. Glinushkin and V. Y. Rud Age and gender recognition from speech signals; Journal of Physics: Conference Series, Volume 1410, 012073, 04. 2019; DOI 10.1088/1742-6596/1410/1/012073.

[51] S. Basu, J. Chakraborty, A. Bag and M. Aftabuddin, "A review on emotion recognition using speech," 2017 International Conference on Inventive Communication and Computational Technologies (ICICCT), Coimbatore, India, 2017, pp. 109-114, doi: 10.1109/ICICCT.2017.7975169.

[52] Sahidullah, Saha, "Design, analysis and experimental evaluation of block based transformation in MFCC computation for speaker recognition", Speech Communication, Volume 54, Issue 4, 2012, Pages 543-565, ISSN 0167-6393, DOI: 10.1016/j.specom.2011.11.004.

## Claims

1. A method for assisting the diagnosis of a bowel disease comprising the steps of:

   a) providing an audio-recording of sounds emitted by the bowel of a subject for the duration of at least one classification time window,
   b) analyzing the processed audio data thereby classifying at least a fraction of the recorded sounds as bowel sound,
   c) computing at least one Mel Frequency Cepstral Coefficient (MFCC)-feature from at least a fraction of the recorded bowel sounds extracted in b),
   d) determining at least one statistical parameter, preferably the mean and/or variance, of at least a fraction of the Mel Frequency Cepstral Coefficient (MFCC)-features computed in c) within at least one classification time window,
   e) using an artificial intelligence (AI) to classify the at least one classification time window as either being indicative for the individual suffering from a bowel disease or for a healthy individual, wherein the AI is taking into account the in step d) determined at least one statistical parameter of at least a fraction of the MFCC-features, thereby predicting the likelihood of a bowel disease for the subject.

2. The method according to any one of the preceding claims, wherein the audio recording within the at least one classification time window is split into audio segments of between 1 and 60 seconds, preferably of about 10 seconds, and/or
the at least one classification time window has a length of between 1 and 60 minutes, preferably of about 10 minutes.

3. The method according to any one of the preceding claims, wherein the duration of the audio recording in step a) exceeds the duration of the at least one classification time window, wherein the classification time window is a sliding classification time window, and wherein in step c) in the sliding classification time window at least one MFCC-feature is computed from at least a fraction of the recorded bowel sounds.

4. The method according to claim 3, wherein the audio recording in step a) is has a duration of over 10 minutes, and wherein the sliding classification time window has a length of 10 minutes.

5. The method according to any one of the preceding claims, wherein the annotation of the bowel sounds in step b) is performed using an artificial intelligence and/or via manual annotation by an expert, preferably wherein the artificial intelligence (AI) in step b) comprises a neural network comprising a convolutional neural network (CNN) architecture.

6. The method according any one of the preceding claims, wherein the recorded audio data is processed by applying at least one filter during and/or after recording, preferably wherein the at least one filter comprises a band-pass filter and/or a recursive linear filter, preferably a high-pass biquadratic filter, more preferably a high-pass biquadratic filter applying a cutoff of 60 Hz.

7. The method according to any one of the preceding claims, wherein in step c) computing at least one MFCC-feature is performed by converting the recorded bowel sounds to a Mel-spectrogram and transforming the Mel-spectrogram using one or more basis functions in order to obtain the at least one MFCC, preferably wherein in step c) the one or more basis functions for transforming the Mel-spectrogram are cosine functions.

8. The method according to the preceding claim, wherein converting the recorded bowel sounds into a Mel-spectrogram is performed by a method comprising the steps of

    i) filtering the bowel sound signal, preferably by adopting a bandpass Butterworth filter,
    ii) conversion of the signal to the frequency domain in order to obtain a frequency spectrum, preferably by performing a Fourier transform on the recorded bowel sound, wherein it is particularly preferred to use a window function, preferably a von Hann or a Hamming window prior to performing the Fourier transform,
    iii) passing the frequency spectrum through a Mel-filterbank to obtain a Mel-spectrogram, wherein the Mel-filterbank is preferably composed of T triangular band pass filters, and a logarithm of the filtered frequency spectrum is taken to obtain a logarithmic energy spectrum.

9. The method according to any one of the preceding claims, wherein in step c) at least 5 MFCC-features, preferably at least 10, are computed.

10. The method according to any one of the preceding claims, wherein the audio-recording in step a) is recorded during one or more different digestive phases of the subject.

11. The method according to the preceding claim, wherein the one or more digestive phases comprise one or more phases of sedentary activities, physical exercise, food intake and/or digestion.

12. The method according to any one of the preceding claims, wherein the audio-recording in step a) is recorded using a wearable recording system comprising one or more microphone(s).

13. The method according to any one of the preceding claims, wherein two or more microphone(s) are used to provide an audio-recording in step a), and wherein the audio data recorded by the two or more microphone(s) is combined for bowel sound extraction in step b) and/or for predicting the likelihood of a bowel disease in step e).

14. Use of the method according to any one of the preceding claims for predicting the likelihood of a bowel disease in a subject.

15. A computer-implemented method or computer program for predicting the likelihood of a bowel disease in a subject, wherein said computer-implemented method or computer program product comprises commands to perform computational steps c)-e) of the method according to any one of claims 1-13.

Fig. 1

**Fig. 2**

**Fig. 3**

**Fig. 3 continued**

(c)

Fig. 4

Fig. 5

**Fig. 6**

Fig. 7

**Bowel Sound (BS) data collection**
- Continuous recording
- Wearable system (e.g. GastroDigitalShirt)

**Data preprocessing**
- Audio filtering
- BS event detection

**BS feature extraction**
- Feature extraction for each BS
- Statistical analysis (mean and variance from BS in 10-minute classification window)

**Automatic IBD detection**
- Classification of each 10-minute classificarion window: Healthy/ IBD

EP 4 578 401 A1

36

| | Europäisches Patentamt<br>European Patent Office<br>Office européen des brevets | **EUROPEAN SEARCH REPORT** | **Application Number**<br><br>EP 23 22 0682 |
|---|---|---|---|

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| X | HUANG YI ET AL: "Fast diagnosis of bowel activities",<br>2017 INTERNATIONAL JOINT CONFERENCE ON NEURAL NETWORKS (IJCNN), IEEE,<br>14 May 2017 (2017-05-14), pages 3042-3049,<br>XP033112424,<br>DOI: 10.1109/IJCNN.2017.7966234<br>[retrieved on 2017-06-30] | 1-6,9-15 | INV.<br>A61B7/00<br>A61B5/00<br>G16H50/20 |
| Y | * section III. METHODOLOGY, A&B&C; section IV. Result; section V. CONCLUSION * | 7,8 | |
| | ----- | | |
| Y | LIU JUZHENG ET AL: "Bowel Sound Detection Based on MFCC Feature and LSTM Neural Network",<br>2018 IEEE BIOMEDICAL CIRCUITS AND SYSTEMS CONFERENCE (BIOCAS), IEEE,<br>17 October 2018 (2018-10-17), pages 1-4,<br>XP033483193,<br>DOI: 10.1109/BIOCAS.2018.8584723<br>[retrieved on 2018-12-20] | 7,8 | |
| | * section II: PRELIMINARY * | | **TECHNICAL FIELDS**<br>**SEARCHED (IPC)**<br><br>A61B<br>G16H |
| | ----- | | |

The present search report has been drawn up for all claims

| Place of search | Date of completion of the search | Examiner |
|---|---|---|
| Munich | 24 May 2024 | Hemb, Björn |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    ..........................................................................
& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019157552 A1, Marshall

**Non-patent literature cited in the description**

- **RONNEBERGER et al.** *Lecture Notes in Computer Science*, 2015, vol. 9351, 234-241
- **SEYEDIAN, S.S.** ; **NOKHOSTIN, F.** ; **MALAMIR, M.D.** A review of the diagnosis, prevention, and treatment methods of inflammatory bowel disease.. *Journal of medicine and life*, 2019, vol. 12 (2), 113
- **BALDASSANO, R.N.** ; **PICCOLI, D.A. et al.** Inflammatory bowel disease in pediatric and adolescent patients. *Gastroenterology Clinics of North America*, 1999, vol. 28 (2), 445-458
- **HAMMER, T.** ; **LANGHOLZ, E.** The epidemiology of inflammatory bowel disease: Balance between East and West? A narrative review. *Digestive Medicine Research*, 2020, vol. 3 (0), https://doi. org/10.21037/dmr-20-149
- **MAASER, C.** ; **STURM, A.** ; **VAVRICKA, S.R.** ; **KUCHARZIK, T.** ; **FIORINO, G.** ; **ANNESE, V.** ; **CALABRESE, E.** ; **BAUMGART, D.C.** ; **BETTEN-WORTH, D.** ; **BORRALHO NUNES, P.** ECCO-ESGAR Guideline for Diagnostic Assessment in IBD Part 1: Initial diagnosis, monitoring of known IBD, detection of complications.. *Journal of Crohn's and Colitis*, 2018, vol. 13 (2), 144-164, https://doi. org/10.1093/ecco-jcc/jjy113 https://academic.oup. com/ecco-jcc/articlepdf/13/2/144/27790815/jjy 113. pdf
- **WAGATSUMA, K.** ; **YOKOYAMA, Y.** ; **NAKASE, H.** Role of biomarkers in the diagnosis and treatment of inflammatory bowel disease.. *Life*, 2021, vol. 11 (12), 1375
- **SHERGILL, A.K.** ; **LIGHTDALE, J.R.** ; **BRUINING, D.H.** ; **ACOSTA, R.D.** ; **CHANDRASEKHARA, V.** ; **CHATHADI, K.V.** ; **DECKER, G.A.** ; **EARLY, D.S.** ; **EVANS, J.A.** ; **FANELLI, R.D. et al.** The role of endoscopy in inflammatory bowel disease. *Gastrointestinal endoscopy*, 2015, vol. 81 (5), 1101-1121
- **KOHLI, A.** ; **HOLZWANGER, E.A.** ; **LEVY, A.N.** Emerging use of artificial intelligence in inflammatory bowel disease. *World Journal of Gastroenterology*, 2020, vol. 26 (44), 6923

- **KILCOYNE, A.** ; **KAPLAN, J.L.** ; **GEE, M.S.** Inflammatory bowel disease imaging: Current practice and future directions. *World Journal of Gastroenterology*, 2016, vol. 22 (3), 917-932, https://doi. org/10.3748/wjg.v22.i3.917
- **STAFFORD, I.S.** ; **GOSINK, M.M.** ; **MOSSOTTO, E.** ; **ENNIS, S.** ; **HAUBEN, M.** A Systematic Review of Artificial Intelligence and Machine Learning Applications to Inflammatory Bowel Disease, with Practical Guidelines for Interpretation. *Inflammatory Bowel Diseases*, 2022, vol. 28 (10), 1573-1583, https://doi. org/10.1093/ ibd/izac115
- **HAN, L.** ; **MACIEJEWSKI, M.** ; **BROCKEL, C.** ; **GORDON, W.** ; **SNAPPER, S.B.** ; **KORZENIK, J.R.** ; **AFZELIUS, L.** ; **ALTMAN, R.B.** A probabilistic pathway score (props) for classification with applications to inflammatory bowel disease. *Bioinformatics*, 2018, vol. 34 (6), 985-993
- **CHIERICI, M.** ; **PUICA, N.** ; **POZZI, M.** ; **CAPIS-TRANO, A.** ; **DONZELLA, M.D.** ; **COLANGELO, A.** ; **OSMANI, V.** ; **JURMAN, G.** Automatically detecting crohn's disease and ulcerative colitis from endoscopic imaging. *BMC Medical Informatics and Decision Making*, 2022, vol. 22 (6), 1-11
- **STIDHAM, R.W.** ; **LIU, W.** ; **BISHU, S.** ; **RICE, M.D.** ; **HIGGINS, P.D.R.** ; **ZHU, J.** ; **NALLAMOTHU, B.K.** ; **WALJEE, A.K.** Performance of a Deep Learning Model vs Human Reviewers in Grading Endoscopic Disease Severity of Patients With Ulcerative Colitis. *JAMA Network Open*, 2019, vol. 2 (5), 193963-193963, https://doi.org/10.1001/jamanetworkopen. 2019.3963
- **FRITZ, D.** ; **WEILITZ, P.B.** Abdominal Assessment. *Home Healthcare Now*, 2016, vol. 34 (3), 151-155, https://doi.org/10.1097/NHH
- **REUBEN, A.** Examination of the abdomen. *Clinical Liver Disease*, 2016, vol. 7 (6), 143-150, https://doi. org/10.1002/cld.556
- **BAID, H.** A critical review of auscultating bowel sounds. *British journal of nursing*, 2009, vol. 18 (18), 1125-1129
- **HUANG, Y.** ; **SONG, I.** ; **RANA, P.** ; **KOH, G.** Fast diagnosis of bowel activities. IEEE, 2017, 3042-3049

- **CRAINE, B.L.** ; **SILPA, M.** ; **O'TOOLE, C.J.** Computerized auscultation applied to irritable bowel syndrome. *Digestive diseases and sciences*, 1999, vol. 44, 1887-1892

- **CRAINE, B.L.** ; **SILPA, M.L.** ; **O'TOOLE, C.J.** Enterotachogram analysis to distinguish irritable bowel syndrome from crohn's disease. *Digestive diseases and sciences*, 2001, vol. 46, 1974-1979

- **RANTA, R.** ; **LOUIS-DORR, V.** ; **HEINRICH, C.** ; **WOLF, D.** ; **GUILLEMIN, F.** Principal component analysis and interpretation of bowel sounds. IEEE, 2004, vol. 3, 227-230

- **DU, X.** ; **ALLWOOD, G.** ; **WEBBERLEY, K.M.** ; **INDERJEETH, A.-J.** ; **OSSEIRAN, A.** ; **MARSHALL, B.J.** Noninvasive diagnosis of irritable bowel syndrome via bowel sound features: Proof of concept. *Clinical and translational gastroenterology*, 2019, vol. 10 (3)

- **SPIEGEL, B.M.** ; **KANESHIRO, M.** ; **RUSSELL, M.M.** ; **LIN, A.** ; **PATEL, A.** ; **TASHJIAN, V.C.** ; **ZEGARSKI, V.** ; **SINGH, D.** ; **COHEN, S.E.** ; **REID, M.W. et al.** Validation of an acoustic gastrointestinal surveillance biosensor for postoperative ileus.. *Journal of Gastrointestinal Surgery*, 2014, vol. 18, 1795-1803

- **CROSS, E.** ; **SAUNDERS, B.** ; **FARMER, A.D.** ; **PRIOR, J.A.** Diagnostic delay in adult inflammatory bowel disease: A systematic review. *Indian Journal of Gastroenterology*, 2023, vol. 42 (1), 40-52, https://doi.org/10.1007/ s12664-022-01303-x

- **KAM, J.** ; **TAYLOR, D.M.** Obesity significantly increases the difficulty of patient management in the emergency department. *Emergency Medicine Australasia*, 2010, vol. 22 (4), 316-323, https://doi. org/10.1111/j.1742-6723. 2010.01307.x

- **ZHAO, Z.** ; **LI, F.** ; **XIE, Y.** ; **WU, Y.** ; **WANG, Y.** BS-Monitor: Noise-Resistant Bowel Sound Monitoring Via Earphones.. *IEEE Transactions on Mobile Computing*, 2023, 1-15, https://doi. org/10.1109/TMC.2023.3270926

- **VERMEIRE, S.** ; **VAN ASSCHE, G.** ; **RUTGEERTS, P.** Laboratory markers in IBD: Useful, magic, or unnecessary toys?. *Gut*, 2006, vol. 55 (3), 426-431, https://doi.org/10.1136/gut.2005.069476

- **BJARNASON, I.** The Use of Fecal Calprotectin in Inflammatory Bowel Disease. *Gastroenterology & Hepatology*, 2017, vol. 13 (1), 53-56

- **GUINDI, M.** ; **RIDDELL, R.H.** Indeterminate colitis.. *Journal of Clinical Pathology*, 2004, vol. 57 (12), 1233-1244, https://doi.org/10.1136/ jcp.2003.015214

- **RANTA, R.** ; **LOUIS-DORR, V.** ; **HEINRICH, C.** ; **WOLF, D.** ; **GUILLEMIN, F.** Towards an acoustic map of abdominal activity. *Proceedings of the 25th Annual International Conference of the IEEE Engineering in Medicine and Biology Society (IEEE Cat. No.03CH37439*, 2003, vol. 3, 2769-27723, https://doi.org/10. 1109/IEMBS.2003.1280491

- **DIMOULAS, C.A.** Audiovisual Spatial-Audio Analysis by Means of Sound Localization and Imaging: A Multimedia Healthcare Framework in Abdominal Sound Mapping.. *IEEE Transactions on Multimedia*, 2016, vol. 18 (10), 1969-1976, https://doi. org/10.1109/TMM. 2016.2594148

- **SAITO, S.-N.** ; **OTSUKA, S.** ; **ZENBUTSU, S.** ; **HORI, S.** ; **HONDA, M.** ; **NAKAGAWA, S.** Generation mechanisms of bowel sounds by simultaneous measurements of X-ray fluoroscopy and bowel sounds. IEEE, 2021, 1593-1596

- **FRIEDMAN, J.H.** Greedy function approximation: a gradient boosting machine. *Annals of statistics*, 2001, 1189-1232

- Gastro Digital Shirt: A Smart Shirt for Digestion Acoustics Monitoring. **BARONETTO, A.** ; **GRAF, L.S.** ; **FISCHER, S.** ; **NEURATH, M.F.** ; **AMFT, O.** ISWC '20: Proceedings of the 2020 International Symposium on Wearable Computers. ACM, 2020, 17-21

- **DIMOULAS, C.A.** ; **PAPANIKOLAOU, G.V.** ; **PETRIDIS, V.** Pattern classification and audiovisual content management techniques using hybrid expert systems: A video-assisted bioacoustics application in Abdominal Sounds pattern analysis. *Expert Systems with Applications*, 2011, vol. 38 (10), 13082-13093, https://doi.org/10.1016/j.eswa.2011.04.115

- **DU, X.** ; **ALLWOOD, G.** ; **WEBBERLEY, K.M.** ; **OSSEIRAN, A.** ; **WAN, W.** ; **VOLIKOVA, A.** ; **MARSHALL, B.J.** A mathematical model of bowel sound generation. *The Journal of the Acoustical Society of America*, 2018, vol. 144 (6), 485-491, https://doi.org/10.1121/1.5080528

- UNet: Convolutional Networks for Biomedical Image Segmentation. **RONNEBERGER, O.** ; **FISCHER, P.** ; **BROX, T.** Medical Image Computing and Computer-Assisted Intervention - MICCAI 2015. Springer International Publishing, 2015, vol. 9351, 234-241

- **TAN, M.** ; **LE, Q.V.** *EfficientNet: Rethinking Model Scaling for Convolutional Neural Networks*, 2019, https://doi.org/10.48550/ arXiv.1905.11946

- **A. BARONETTO** ; **L. S. GRAF** ; **S. FISCHER** ; **M. F. NEURATH** ; **O. AMFT**. Segment-based Spotting of Bowel Sounds using Pretrained Models in Continuous Data Streams. *IEEE Journal of Biomedical and Health Informatics*, July 2023, vol. 27 (7), 3164-3174

- **GONG, Y.** ; **CHUNG, Y.-A.** ; **GLASS, J.** *PSLA: Improving Audio Tagging with Pretraining, Sampling, Labeling, and Aggregation*, 2021, https://doi.org/10.1109/TASLP. 2021.3120633
- Audio Set: An ontology and human-labeled dataset for audio events. **GEMMEKE, J.F.** ; **ELLIS, D.P.W.** ; **FREEDMAN, D.** ; **JANSEN, A.** ; **LAWRENCE, W.** ; **MOORE, R.C.** ; **PLAKAL, M.** ; **RITTER, M.** 2017 IEEE International Conference on Acoustics, Speech and Signal Processing (ICASSP). IEEE, 2017, 776-780
- **HE, K.** ; **ZHANG, X.** ; **REN, S.** ; **SUN, J.** *Delving Deep into Rectifiers: Surpassing Human Level Performance on ImageNet Classification*, 2015, https://doi.org/10.48550/ arXiv.1502.01852
- **KINGMA, D.P.** ; **BA, J.** *Adam: A Method for Stochastic Optimization*, 2014, https: //doi.org/10.48550/arXiv.1412.6980
- **PARK, D.S.** ; **CHAN, W.** ; **ZHANG, Y.** ; **CHIU, C.-C.** ; **ZOPH, B.** ; **CUBUK, E.D.** ; **LE, Q.V.** *SpecAugment: A Simple Data Augmentation Method for Automatic Speech Recognition*, 2019, https: //doi.org/10.21437/Interspeech.2019-2680
- **ROSS, B.C.** Mutual information between discrete and continuous data sets. *PloS one*, 2014, vol. 9 (2), 87357
- **GOUYON, F.** ; **HERRERA, P.** Exploration of techniques for automatic labeling of audio drum tracks instruments. *Proceedings of MOSART: Workshop on Current Directions in Computer Music*, 2001
- **PLOMP, R.** ; **LEVELT, W.J.M.** Tonal consonance and critical bandwidth. *The journal of the Acoustical Society of America*, 1965, vol. 38 (4), 548-560
- **DE CHEVEIGNÉ, A.** ; **KAWAHARA, H.** Yin, a fundamental frequency estimator for speech and music.. *The Journal of the Acoustical Society of America*, 2002, vol. 111 (4), 1917-1930
- *Digestive-system 3D Model by Genesis1*, https://sketchfab.com/models/ 2db2c9090e1 d4e1891826958398d324f
- **J. FICEK** ; **K. RADZIKOWSKI** ; **J. K. NOWAK** ; **O. YOSHIE** ; **J. WALKOWIAK** ; **R. NOWAK**. Analysis of Gastrointestinal Acoustic Activity Using Deep Neural Networks. *Sensors*, November 2021, vol. 21 (22)
- **MAHALAKSHMI, P.** A Review On Voice Activity Detection And Mel-Frequency Cepstral Coefficients For Speaker Recognition (Trend Analysis).. *Asian Journal of Pharmaceutical and Clinical Research*, 2016, vol. 9, 360
- **A. ABDULSATAR** ; **V. DAVYDOV** ; **V. YUSHKOVA** ; **A. P. GLINUSHKIN** ; **V. Y. RUD**. Age and gender recognition from speech signals. *Journal of Physics: Conference Series*, 2019, vol. 1410, 012073
- **S. BASU** ; **J. CHAKRABORTY** ; **A. BAG** ; **M. AFTABUDDIN**. A review on emotion recognition using speech. *2017 International Conference on Inventive Communication and Computational Technologies (ICICCT), Coimbatore, India*, 2017, 109-114
- **SAHIDULLAH, SAHA**. Design, analysis and experimental evaluation of block based transformation in MFCC computation for speaker recognition. *Speech Communication*, 2012, vol. 54 (4), ISSN 0167-6393, 543-565